# EUROPEAN PATENT APPLICATION

(11) **EP 4 840 453 A1**
(43) Date of publication of application: **18.11.2026**
(21) Application number: 25738938.7
(22) Date of filing: 10.01.2025
(51) Int. Cl.: A61K 31/53, A61K 31/436, A61K 31/497, A61K 31/506, A61K 31/513, A61K 31/517, A61K 31/519, A61K 31/5377, A61K 31/573, A61K 31/664, A61K 39/395, A61K 45/00, A61P 35/00, A61P 35/02, A61P 43/00

(54) **CANCER TREATMENT AGENT CONTAINING CLK INHIBITOR AS ACTIVE INGREDIENT**

(30) Priority: 12.01.2024 JP 2024003374
(71) Applicant: Chordia Therapeutics Inc., Fujisawa-shi, Kanagawa 251-0012 (JP)
(72) Inventor: MORISHITA Daisuke, Fujisawa-shi, Kanagawa 251-0012 (JP); SATOH Yoshihiko, Fujisawa-shi, Kanagawa 251-0012 (JP); MIZUTANI Akio, Fujisawa-shi, Kanagawa 251-0012 (JP)
(74) Representative: Weickmann & Weickmann PartmbB
(86) International application number: PCT/JP2025/000724
(87) International publication number: WO 2025/150571

(57) **Abstract**

It is an object of the present invention to find a combination of agents useful for the prevention or the treatment of cancer or a precancerous lesion, and to provide such a combination of agents as a pharmaceutical composition.

The object is solved by a combination agent including, in combination, a first ingredient that is a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 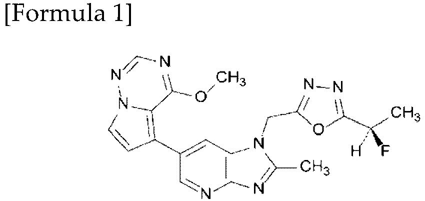 or a salt or a hydrate thereof, and a second ingredient containing at least one antitumor agent.

## Description

### Technical Field

The present invention relates to a pharmaceutical composition for preventing or treating cancer or a precancerous lesion, the composition containing, as an active ingredient, 1-((5-(1R)-1-fluoroethyl)-1,3,4-oxadiazolyl)methyl)-6-(4-methoxypyrrolo[2,1-f][1,2,4]triazin-5-yl)-2-methyl-1H-imidazo[4,5-b]pyridine represented by the following formula (I): 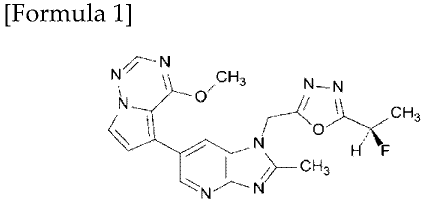 (hereinafter also referred to as "Compound A") or a derivative thereof.

### Background Art

Abnormal regulation of alternative splicing mechanisms has been reported in various diseases such as neurodegenerative diseases, amyotrophic lateral sclerosis, and cancer. Particularly in cancer, it has been revealed that a splicing variant specific for cancer, which is produced by abnormal alternative splicing plays an important role in the survival and the invasion of cancer. In recent years, it has also been clarified that, in myelodysplastic syndrome, a spliceosome ingredient such as SF3B1, SRSF2, and U2AF1 is mutated highly frequently. These findings indicate that regulation of the alternative splicing mechanism plays an important role in cancer.

The CLK family kinases are a type of protein kinase with dual specificity that possess both a serine/threonine kinase activity and a tyrosine kinase activity, and there are four kinases, that is, from CLK1 to CLK4. The CLK controls the localization of an SR protein such as SRSF1 by phosphorylating them, and thus regulates the splicing regulatory mechanism mediated by the SR protein. It has been clarified that by modulating the alternative splicing through inhibition of the CLK kinase activity, a signal essential for the survival of cancer can be blocked, and thus the proliferation of the cancer cells can be inhibited. It has also been clarified that the CLK2 functions as an oncogene in breast cancer.

Therefore, it is considered that the inhibition of the CLK kinase activity can be a promising method for treatment of cancer.

Patent Literature 1 discloses a variety of compounds that have an inhibitory effect on the cdc2-like kinase (hereinafter also referred to as the "CLK"), which include the compound A.

### Document List

### Patent Literature

Patent Literature 1: WO 2017/188374

### Summary of Invention

### Technical Problem

One of the objects of the present invention is to find combinations of agents useful for the prevention or the treatment of cancer or a precancerous lesion, and to provide such combination of agents as a pharmaceutical composition to be administered simultaneously or sequentially.

### Solution to Problem

The present inventors found that a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 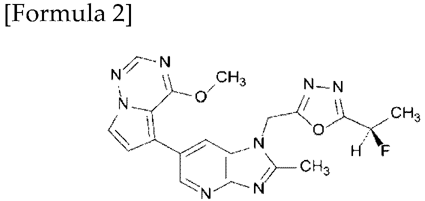 or a derivative thereof attains an antitumor effect significantly higher when used in combination with an antitumor agent, in comparison with the case where the CLK inhibitor is used alone, and thus the combination may be useful for the prevention and/or the treatment of cancer or a precancerous lesion. Based on this finding, the present inventors conducted extensive studies and completed the present invention. In other words, the present invention is as follows.
[1] A combination agent for preventing or treating cancer or a precancerous lesion, the agent including, in combination, a first ingredient that is a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 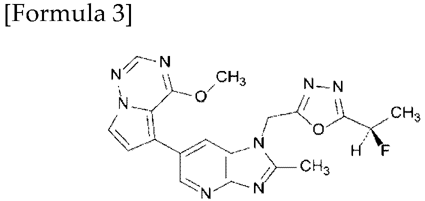 or a salt or a hydrate thereof, and a second ingredient containing at least one antitumor agent.
[2] The combination agent according to [1], wherein the first ingredient and the second ingredient are administered simultaneously or sequentially.
[3] The combination agent according to [1] or [2], wherein the antitumor agent of the second ingredient is selected from the group consisting of a hormonal therapeutic agent, a chemotherapeutic agent, an immunotherapeutic agent, and a molecular targeted agent.
[4] The combination agent according to any one of [1] to [3], wherein the antitumor agent of the second ingredient is selected from the group consisting of an antimetabolic agent, a CDK inhibitor, an anticancer antibiotic, a proteasome inhibitor, a topoisomerase I inhibitor, an ALK inhibitor, an alkylating agent, a PI3K inhibitor, an adrenocortical hormone agent, a plant-derived anticancer agent, an mTOR inhibitor, a BCL-2 inhibitor, an Akt inhibitor, a HER2 inhibitor, an EGF receptor inhibitor, a PARP inhibitor, a CSF inhibitor, a FLT3 inhibitor, an Aurora inhibitor, a MEK (MEK1/2) inhibitor, a Raf inhibitor, a PLK inhibitor, a NEDD8 inhibitor, a UAE inhibitor, a differentiation inducing agent, a cytotoxic agent, an IDH1/2 inhibitor, a VEGFR inhibitor, and an anti-PD-1 antibody.
[5] The combination agent according to any one of [1] to [4], wherein the antitumor agent of the second ingredient is at least one antitumor agent selected from the group consisting of 5-fluorouracil, abemaciclib, actinomycin D, all-trans retinoic acid, AZD6738, BAY1895344, bortezomib, camptothecin, ceritinib, crizotinib, cyclophosphamide, cytarabine, dactolisib, dasatinib, decitabine, dexamethasone, dinaciclib, docetaxel, doxorubicin, duvelisib, entinostat, epirubicin, etoposide, everolimus, gemcitabine, ibrutinib, imatinib, ipatasertib, tucatinib, KU-60019, lapatinib, masitinib, mercaptopurine, methotrexate, mitomycin C, mitoxantrone, MK-1775, momelotinib, navitoclax, niraparib, olaparib, palbociclib, pexidartinib, PHA-793887, quizartinib, ruxolitinib, SCH900776, SN-38, topotecan, tozasertib, trametinib, UMI-77, VE-822, vemurafenib, venetoclax, vinblastine, vincristine, volasertib, cisplatin, melphalan, oxaliplatin, EPZ-6438, JQ1, MLN-4924, MLN-7243, MPI-0479605, NMS-P715, carboplatin, paclitaxel, azacitidine, hydroxyurea, ivosidenib, enasidenib, gilteritinib, sorafenib, lenvatinib, regorafenib, midostaurin, and rucaparib.
[6] The combination agent according to any one of [1] to [4], wherein the antitumor agent of the second ingredient is an anti-PD-1 antibody.
[7] The combination agent according to any one of [1] to [6], wherein the CLK-related disease is selected from the group consisting of colorectal cancer, lung cancer, mesothelioma, pancreatic cancer, pharyngeal cancer, laryngeal cancer, esophageal cancer, gastric cancer, duodenal cancer, small intestinal cancer, breast cancer, ovarian cancer, testicular tumor, prostate cancer, liver cancer, thyroid cancer, kidney cancer, uterine cancer, ovarian cancer, gestational trophoblastic cancer, brain tumor, retinoblastoma, skin cancer, sarcoma, malignant bone tumor, bladder cancer, acute myeloid leukemia, myelodysplastic syndrome, and other hematologic cancers.
[8] The combination agent according to any one of [1] to [7], wherein the combination agent is used for preventing or treating myelodysplastic syndrome, acute myeloid leukemia, or ovarian cancer.
[9] A pharmaceutical composition for preventing or treating cancer or a precancerous lesion, the composition including a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 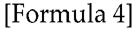 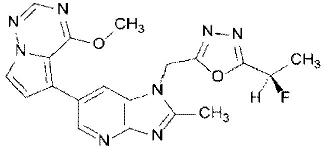 or a salt or a hydrate thereof, wherein the pharmaceutical composition is administered to a patient in need thereof simultaneously or sequentially in combination with an antitumor agent.
[10] A pharmaceutical composition for preventing or treating cancer or a precancerous lesion, the composition including an antitumor agent, and the pharmaceutical composition being administered to a patient in need thereof simultaneously or sequentially in combination with a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 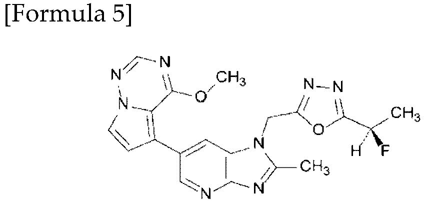 or a salt or a hydrate thereof.
[11] A method for treating cancer or a precancerous lesion, the method including administering to a patient in need of a treatment of cancer or a precancerous lesion, simultaneously or sequentially, an effective amount of a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 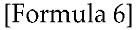 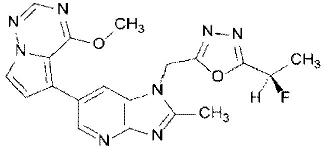 or a salt or a hydrate thereof, and an effective amount of an antitumor agent.
[12] A method for treating cancer or a precancerous lesion, the method comprising administering to a patient in need of a treatment of cancer an effective amount of a CLK inhibitor containing 1-((5-(1R)-1-fluoroethyl)-1,3,4-oxadiazolyl)methyl)-6-(4-methoxypyrrolo[2,1-f][1,2,4]triazin-5-yl)-2-methyl-1H-imidazo[4,5-b]pyridine represented by the following formula (I): 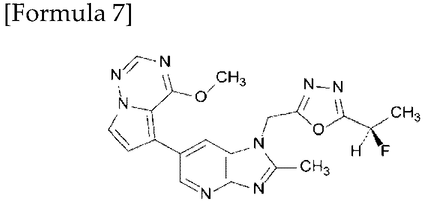 or a salt or a hydrate thereof, wherein the method further comprises administering an effective amount of an antitumor agent.
[13] Use of a CLK inhibitor containing 1-((5-(1R)-1-fluoroethyl)-1,3,4-oxadiazolyl)methyl)-6-(4-methoxypyrrolo[2,1-f][1,2,4]triazin-5-yl)-2-methyl-1H-imidazo[4,5-b]pyridine represented by the following formula (I): 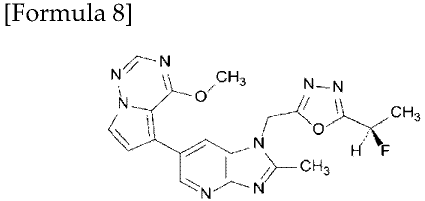 or a salt or a hydrate thereof, for manufacturing a therapeutic agent for cancer or a precancerous lesion, wherein the CLK inhibitor is administered in combination with an antitumor agent.
[14] Use of an antitumor agent for manufacturing a therapeutic agent for cancer or a precancerous lesion, wherein the antitumor agent is administered in combination with a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 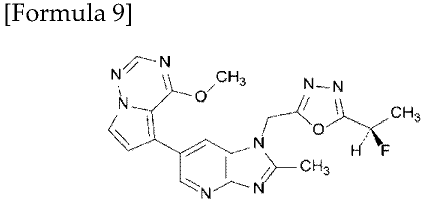 or a salt or a hydrate thereof.
[15] A combination agent for preventing or treating myelodysplastic syndrome, acute myeloid leukemia, or a precancerous lesion thereof, the agent including, in combination, a first ingredient that is a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 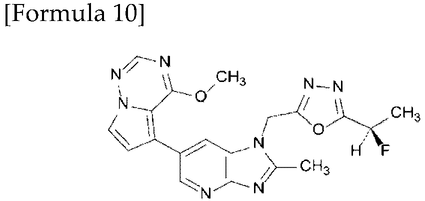 or a salt or a hydrate thereof, and a second ingredient containing at least one antitumor agent selected from the group consisting of quizartinib, midostaurin, cytarabine, venetoclax, and azacitidine.
[16] A combination agent for preventing or treating myelodysplastic syndrome, acute myeloid leukemia, or a precancerous lesion thereof, the agent including, in combination, a first ingredient that is a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 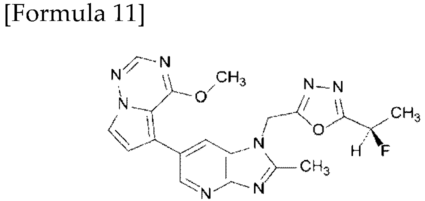 or a salt or a hydrate thereof, and a second ingredient containing an antitumor agent of venetoclax and/or azacitidine.
[17] A combination agent for preventing or treating ovarian cancer or a precancerous lesion thereof, the agent comprising, in combination, a first ingredient that is a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 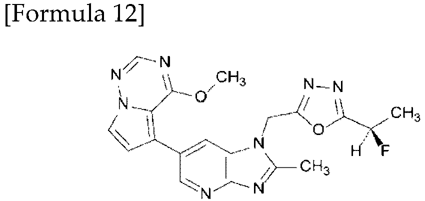 or a salt or a hydrate thereof, and a second ingredient containing at least one antitumor agent selected from the group consisting of niraparib, docetaxel, carboplatin, and paclitaxel.

### Effects of Invention

The combination according to the present invention is useful for the prevention and/or the treatment of cancer.

### Brief Description of Drawings

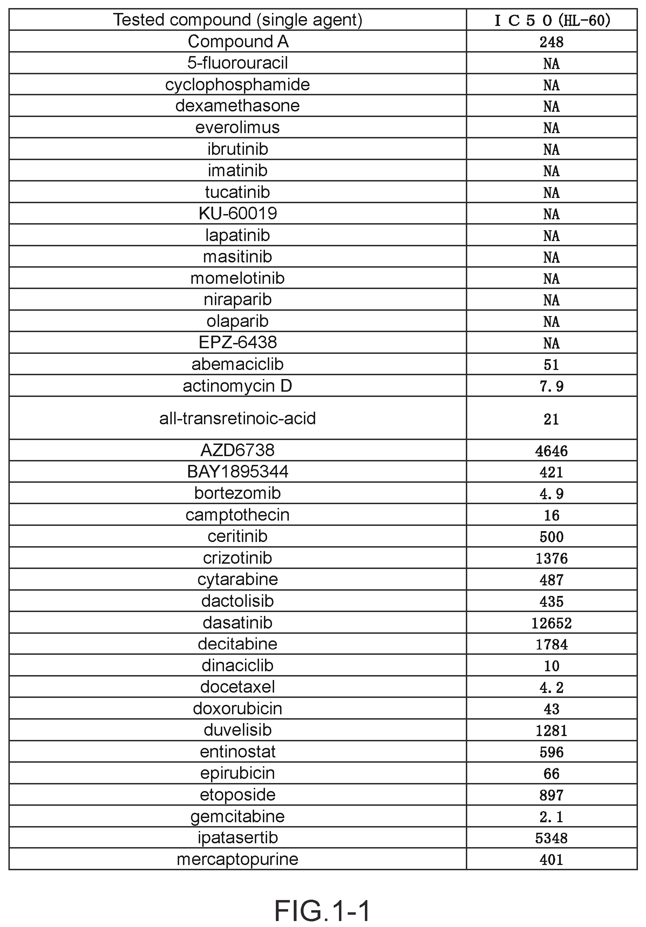

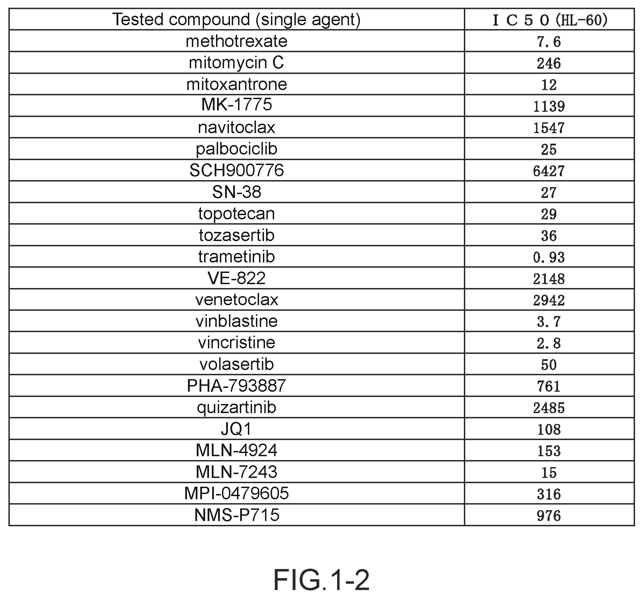

[Fig. 1-1] A drawing illustrating IC50 values of respective compounds (single agents) in HL-60.
[Fig. 1-2] A drawing illustrating IC50 values of respective compounds (single agents) in HL-60.
[Fig. 2-1] A drawing illustrating IC50 values of respective compounds (single agents) in NCI-H661.
[Fig. 2-2] A drawing illustrating IC50 values of respective compounds (single agents) in NCI-H661.
[Fig. 3] A drawing illustrating IC50 values of respective compounds (single agents) in MV-4-11.
[Fig. 4] A drawing illustrating changes in tumor volume after administration of each agent in a model with subcutaneous transplantation of MC38 cells.
[Fig. 5] A drawing illustrating changes in body weight after administration of each agent in the model with subcutaneous transplantation of the MC38 cells.
[Fig. 6] A drawing illustrating changes in tumor volume after administration of each agent in a model with subcutaneous transplantation of MV-4-11 cells.
[Fig. 7] A drawing illustrating changes in body weight after administration of each agent in the model with subcutaneous transplantation of the MV-4-11 cells.
[Fig. 8] A drawing illustrating changes in tumor volume after administration of each agent in a model with subcutaneous transplantation of MV-4-11 cells.
[Fig. 9] A drawing illustrating changes in body weight after administration of each agent in the model with subcutaneous transplantation of the MV-4-11 cells.
[Fig. 10] A drawing illustrating changes in tumor volume after administration of each agent in a model with subcutaneous transplantation of OVCAR-8 cells.
[Fig. 11] A drawing illustrating changes in body weight after administration of each agent in the model with subcutaneous transplantation of the OVCAR-8 cells.

### Description of Embodiments

Hereinafter, detailed description will be provided with regard to combinations included in embodiments of the present invention, particularly in terms of a combination agent including, in combination, a CLK inhibitor containing Compound A or a derivative thereof and an antitumor agent; a pharmaceutical composition including a CLK inhibitor containing the Compound A or a derivative thereof for use in combination with the antitumor agent; a commercial package including a combination of pharmaceutical compositions of the CLK inhibitor containing the Compound A or a derivative thereof and the antitumor agent; and further, a method for preventing and/or treating cancer or a precancerous lesion, including administering the combination agent, the pharmaceutical composition, or the combination of the pharmaceutical compositions to a patient in need thereof.

### (1) Compound A

Within the scope of the present invention, "Compound A" refers to 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I):

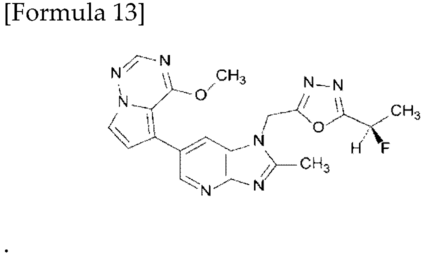

Further, within the scope of the present invention, the derivative of the Compound A includes salts and hydrates of the Compound A, or optical isomers, tautomers, solvates, and the like thereof. Moreover, such a derivative of the Compound A may also be a crystal or a clathrate, and the crystal form may be a single crystal form, a mixture of crystal forms, a co-crystal, or the like.

As the salt of the Compound A, a pharmacologically acceptable salt is preferred, and examples thereof include a salt with an inorganic base, a salt with an organic base, a salt with an inorganic acid, a salt with an organic acid, and a salt with a basic or acidic amino acid. Preferred examples of the salt with an inorganic base include alkali metal salts such as sodium salts and potassium salts; alkaline earth metal salts such as calcium salts and magnesium salts; and aluminum salts and ammonium salts. Preferred examples of the salt with an organic base include salts with trimethylamine, triethylamine, pyridine, picoline, ethanolamine, diethanolamine, triethanolamine, tromethamine [tris(hydroxymethyl)methylamine], tert-butylamine, cyclohexylamine, benzylamine, dicyclohexylamine, and N,N-dibenzylethylenediamine.

Preferred examples of the salt with an inorganic acid include salts with hydrochloric acid, hydrobromic acid, nitric acid, sulfuric acid, and phosphoric acid.

Preferred examples of the salt with an organic acid include salts with formic acid, acetic acid, trifluoroacetic acid, phthalic acid, fumaric acid, oxalic acid, tartaric acid, maleic acid, citric acid, succinic acid, malic acid, methanesulfonic acid, benzenesulfonic acid, and p-toluenesulfonic acid. Preferred examples of the salt with a basic amino acid include salts with arginine, lysine, and ornithine.

Preferred examples of the salt with an acidic amino acid include salts with aspartic acid and glutamic acid.

Further, details of the method for producing the Compound A or the derivative thereof are disclosed in WO 2017/188374, the contents of which are incorporated herein by reference.

### (2) Antitumor Agent

The "antitumor agent" used in the combination of the pharmaceutical compositions according to the present invention may be at least one antitumor agent selected from the group consisting of hormone therapeutic agents, chemotherapeutic agents, immunotherapeutic agents, and molecular targeted agents including agents that inhibit the action of cell growth factors and their receptors, and may be, for example, a combination of at least two or at least three antitumor agents selected from the above.

Examples of the hormone therapeutic agent that can be used include phosphestrol, diethylstilbestrol, chlorotrianisene, medroxyprogesterone acetate, megestrol acetate, chlormadinone acetate, cyproterone acetate, danazol, allylestrenol, gestrinone, mepartricin, raloxifene, ormeloxifene, levormeloxifene, antiestrogens (for example, tamoxifen citrate and toremifene citrate), oral contraceptive preparations, mepitiostane, testololactone, aminoglutethimide, LH-RH agonists (for example, goserelin acetate, buserelin, and leuprorelin acetate), droloxifene, epitiostanol, ethinyl estradiol sulfonate, aromatase inhibitors (for example, fadrozole hydrochloride, anastrozole, letrozole, exemestane, vorozole, and formestane), antiandrogens (for example, flutamide, bicalutamide, nilutamide, and enzalutamide), 5α-reductase inhibitors (for example, finasteride, epristeride, and dutasteride), adrenocortical hormone-based agents (for example, dexamethasone, prednisolone, betamethasone, and triamcinolone), androgen biosynthesis inhibitors (for example, abiraterone), retinoids, agents that delay metabolism of retinoid (for example, liarozole), thyroid hormones, and DDS formulations thereof.

Examples of the chemotherapeutic agent that can be used include alkylating agents, antimetabolic agents, anticancer antibiotics, and plant-derived anticancer agents.

Examples of the alkylating agent that can be used include nitrogen mustard, nitrogen mustard-N-oxide hydrochloride, chlorambucil, cyclophosphamide, ifosfamide, thiotepa, carboquone, improsulfan tosylate, busulfan, nimustine hydrochloride, mitobronitol, melphalan, dacarbazine, ranimustine, estramustine phosphate sodium, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, bendamustine, temozolomide, treosulfan, trofosfamide, zinostatin stimalamer, adozelesin, systemustine, bizelesin, and DDS formulations thereof.

Examples of the antimetabolic agents that can be used include mercaptopurine, 6-mercaptopurine riboside, thioinosine, methotrexate, pemetrexed, enocitabine, cytarabine, cytarabine ocfosfate, ancitabine hydrochloride, 5-FU agents (for example, fluorouracil, tegafur, UFT, doxifluridine, carmofur, galocitabine, emitefur, and capecitabine), aminopterin, nelzarabine, leucovorin calcium, thioguanine, butocin, calcium folinate, calcium levofolinate, cladribine, fludarabine, gemcitabine, pentostatin, piritrexim, idoxuridine, mitoguazone, tiazofurin, ambamustine, bendamustine, hydroxyurea, and DDS formulations thereof.

Examples of the anticancer antibiotics that can be used include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, zarcomycin, carzinophilin, mitotane, zorubicin hydrochloride, mitoxantrone hydrochloride, idarubicin hydrochloride, and DDS formulations thereof (for example, PEG liposome-encapsulated doxorubicin).

Examples of the plant-derived anticancer agent that can be used include etoposide, etoposide phosphate, vinblastine sulfate, vincristine sulfate, vindesine sulfate, teniposide, paclitaxel, docetaxel, cabazitaxel, vinorelbine, and DDS formulations thereof.

Examples of the immunotherapeutic agent that can be used include picibanil, krestin, sizofiran, lentinan, ubenimex, interferon, interleukin, macrophage colony-stimulating factor, granulocyte colony-stimulating factor, erythropoietin, lymphotoxin, BCG vaccine, Corynebacterium parvum, levamisole, polysaccharide K, procodazole, anti-CTLA4 antibodies (for example, ipilimumab and tremelimumab), anti-PD-1 antibodies (for example, nivolumab and pembrolizumab), and anti-PD-L1 antibodies.

Examples of the molecular targeted agent include agents that inhibit the action of cell growth factors and their receptors, BCL-2 inhibitors, PARP inhibitors, and the like. Here, the cell growth factor may be any substance that promotes cell proliferation, and generally includes factors that are peptides having a molecular weight of 20,000 or less and exert their effects at low concentrations through binding to receptors. Specific examples of such a substance that can be used include (i) EGF (epidermal growth factor) or substances having substantially the same activity thereas, for example, TGFα; (ii) insulin or substances having substantially the same activity thereas, for example, insulin and IGF (insulin-like growth factor; IGF-1 and IGF-2); (iii) FGF (fibroblast growth factor) or substances having substantially the same activity thereas, for example, acidic FGF, basic FGF, KGF (keratinocyte growth factor), and FGF-10; and (iv) other cell growth factors, for example, CSF (colony-stimulating factor), EPO (erythropoietin), IL-2 (interleukin-2), NGF (nerve growth factor), PDGF (platelet-derived growth factor), TGFβ (transforming growth factor β), HGF (hepatocyte growth factor), VEGF (vascular endothelial growth factor), heregulin, and angiopoietin.

Examples of the receptor for a cell growth factor include any receptor as long as it has the ability to bind to the above cell growth factors, and specific examples of such a receptor that can be used include an EGF receptor, a heregulin receptor, for example, HER3, an insulin receptor, an IGF-1 receptor, an IGF-2 receptor, a FGF-1 receptor or a FGF-2 receptor, a VEGF receptor, an angiopoietin receptor, for example, Tie2, a PDGF receptor, and the like.

Accordingly, examples of agents that inhibit the action of cell growth factors and their receptors that can be used include EGF inhibitors, TGFα inhibitors, heregulin inhibitors, insulin inhibitors, IGF inhibitors, FGF inhibitors, KGF inhibitors, CSF inhibitors, EPO inhibitors, IL-2 inhibitors, NGF inhibitors, PDGF inhibitors, TGFβ inhibitors, HGF inhibitors, VEGF inhibitors, angiopoietin inhibitors, EGFR inhibitors, HER2 inhibitors, HER4 inhibitors, insulin receptor inhibitors, IGF-1 receptor inhibitors, IGF-2 receptor inhibitors, FGFR 1 inhibitors, FGFR 2 inhibitors, FGFR 3 inhibitors, FGF 4 inhibitors, VEGF receptor inhibitors (VEGFR inhibitors), Tie-2 inhibitors, PDGF receptor inhibitors, Abl inhibitors, Raf inhibitors, FLT3 inhibitors, c-Kit inhibitors, Src inhibitors, PKC inhibitors, Smo inhibitors, ALK inhibitors, ROR1 inhibitors, Trk inhibitors, Ret inhibitors, mTOR inhibitors, Aurora inhibitors, PLK inhibitors, MEK (MEK1/2) inhibitors, MET inhibitors, CDK inhibitors, Akt inhibitors, ERK inhibitors, PI3K inhibitors, and the like. More specifically, anti-VEGF antibodies (for example, Bevacizumab, Ramucirumab, and Aflibercept), anti-HER2 antibodies (for example, Trastuzumab and Pertuzumab), anti-EGFR antibodies (for example, Cetuximab, Panitumumab, Matuzumab, and Nimotuzumab), anti-HGF antibodies, Imatinib, Erlotinib, Gefitinib, Sorafenib, Sunitinib, Dasatinib, Lapatinib, Vatalanib, Ibrutinib, Bosutinib, Cabozantinib, Crizotinib, Alectinib, Vismodegib, Axitinib, Motesanib, Nilotinib, 6-[4-(4-ethylpiperazin-1-ylmethyl)phenyl]-N-[1(R)-phenylethyl]-7H-pyrrolo[2,3-d]pyrimidin-4-amine (AEE-788), Vandetanib, Temsirolimus, Everolimus, Enzastaurin, Tozasertib, 2-[N-[3-[4-[5-[N-(3-fluorophenyl)carbamoylmethyl]-1H-pyrazol-3-ylamino]quinazolin-7-yloxy]propyl]-N-ethylamino]ethyl phosphate ester (AZD-1152), 4-[9-chloro-7-(2,6-difluorophenyl)-5H-pyrimido[5,4-d][2]benzazepin-2-ylamino]benzoic acid, N-[2-methoxy-5-[(E)-2-(2,4,6-trimethoxyphenyl)vinylsulfonylmethyl]phenyl]glycine sodium salt (ON-1910Na), Volasertib, Selumetinib, Trametinib, N-[2(R),3-dihydroxypropoxy]-3,4-difluoro-2-(2-fluoro-4-iodophenylamino)benzamide (PD-0325901), Bosutinib, Regorafenib, Afatinib, Idelalisib, Ceritinib, Dabrafenib, anti-CD47 antibodies (for example, Magrolimab), anti-CD33/CD2 antibodies (for example, BiTE), anti-CD123/CD3 antibodies (for example, Flotetuzumab), folate receptor ADC (for example, Mirvetuximab soravtansine), menin-MLL binding inhibitors (for example, Ziftmenib, Revumenib, DS-1594b, and JNJ-75276617), VEGFR inhibitors (for example, Cediranib), and the like may be used.

In one embodiment of the present invention, the antitumor agent selected from the group consisting of an antimetabolic agent, a CDK inhibitor, an anticancer antibiotic, a proteasome inhibitor, a topoisomerase I inhibitor, an ALK inhibitor, an alkylating agent, a PI3K inhibitor, an adrenocortical hormone agent, a plant-derived anticancer agent, an mTOR inhibitor, a BCL-2 inhibitor, an Akt inhibitor, a HER2 inhibitor, an EGFR inhibitor, a PARP inhibitor, a CSF inhibitor, a FLT3 inhibitor, an Aurora inhibitor, a MEK (MEK1/2) inhibitor, a Raf inhibitor, a PLK inhibitor, a NEDD8 inhibitor, a UAE inhibitor, a differentiation-inducing agent, a cytotoxic agent, an IDH1/2 inhibitor, a VEGFR inhibitor, and an anti-PD-1 antibody may be used.

In a preferred embodiment of the present invention, in detail, the antitumor agent may be at least one antitumor agent selected from the group consisting of 5-fluorouracil, abemaciclib, actinomycin D, all-trans retinoic acid, AZD6738, BAY1895344, bortezomib, camptothecin, ceritinib, crizotinib, cyclophosphamide, cytarabine, dactolisib, dasatinib, decitabine, dexamethasone, dinaciclib, docetaxel, doxorubicin, duvelisib, entinostat, epirubicin, etoposide, everolimus, gemcitabine, ibrutinib, imatinib, ipatasertib, tucatinib, KU-60019, lapatinib, masitinib, mercaptopurine, methotrexate, mitomycin C, mitoxantrone, MK-1775, momelotinib, navitoclax, niraparib, olaparib, palbociclib, pexidartinib, PHA-793887, quizartinib, ruxolitinib, SCH900776, SN-38, topotecan, tozasertib, trametinib, UMI-77, VE-822, vemurafenib, venetoclax, vinblastine, vincristine, volasertib, cisplatin, melphalan, oxaliplatin, EPZ-6438, JQ1, MLN-4924, MLN-7243, MPI-0479605, NMS-P715, carboplatin, paclitaxel, azacitidine, hydroxyurea, ivosidenib, enasidenib, gilteritinib, sorafenib, lenvatinib, regorafenib, midostaurin, and rucaparib.

In a particular embodiment of the present invention, the antitumor agent may be at least one antitumor agent selected from the group consisting of quizartinib, midostaurin, cytarabine, venetoclax, and azacitidine.

Further, in a particular embodiment of the present invention, the antitumor agent may be at least one antitumor agent selected from the group consisting of niraparib, docetaxel, carboplatin, and paclitaxel.

Further, in a preferred embodiment of the present invention, the antitumor agent may be an anti-PD-1 antibody.

### (3) Indications

The pharmaceutical composition of the present invention exhibits a selective and excellent inhibitory activity against CLK, by combining the CLK inhibitor containing the Compound A or a derivative thereof with the antitumor agent, and is also excellent in terms of expression of the pharmacological effect, pharmacokinetics (for example, absorption, distribution, metabolism, and excretion), solubility (for example, water solubility), interaction with other agents (for example, drug metabolizing enzyme inhibitory action), safety (for example, acute toxicity, chronic toxicity, genotoxicity, reproductive toxicity, cardiotoxicity, carcinogenicity, and central toxicity), and stability (for example, chemical stability and stability against enzymes), and is therefore useful as a drug.

Accordingly, the pharmaceutical composition of the present invention can be used as a drug for inhibiting excessive (abnormal) CLK action in mammals (for example, mice, rats, hamsters, rabbits, cats, dogs, cattle, sheep, monkeys, and humans).

Further, the pharmaceutical composition of the present invention can be used as a drug for the prevention and/or the treatment of cancer or a precancerous lesion. Here, the cancer or the precancerous lesion includes a CLK-related disease. Within the scope of the present invention, the CLK-related disease refers to a disease that may be affected by CLK. Further, the cancer or the precancerous lesion includes, for example, colorectal cancer (for example, colon cancer, rectal cancer, anal cancer, familial colorectal cancer, hereditary non-polyposis colorectal cancer, and gastrointestinal stromal tumor), lung cancer (for example, non-small cell lung cancer, small cell lung cancer, and malignant mesothelioma), mesothelioma, pancreatic cancer (for example, pancreatic ductal cancer and pancreatic endocrine tumor), pharyngeal cancer, laryngeal cancer, esophageal cancer, gastric cancer (for example, papillary adenocarcinoma, mucinous adenocarcinoma, and adenosquamous carcinoma), duodenal cancer, small intestinal cancer, breast cancer (for example, invasive ductal carcinoma, non-invasive ductal carcinoma, and inflammatory breast cancer), ovarian cancer (for example, epithelial ovarian cancer, extragonadal germ cell tumor, ovarian germ cell tumor, and ovarian low malignant potential tumor), testicular tumor, prostate cancer (for example, hormone-dependent prostate cancer, hormone-independent prostate cancer, and castration-resistant prostate cancer), liver cancer (for example, hepatocellular carcinoma, primary liver cancer, and extrahepatic bile duct cancer), thyroid cancer (for example, medullary thyroid cancer), kidney cancer (for example, renal cell carcinoma, for example, clear-cell renal cell carcinoma, and transitional cell carcinoma of the renal pelvis and ureter), uterine cancer (for example, cervical cancer, endometrial cancer, and uterine sarcoma), gestational choriocarcinoma, brain tumor (for example, medulloblastoma, glioma, pineal astrocytic tumor, pilocytic astrocytoma, diffuse astrocytoma, anaplastic astrocytoma, and pituitary adenoma), retinoblastoma, skin cancer (for example, basal cell carcinoma and malignant melanoma), sarcoma (for example, rhabdomyosarcoma, leiomyomatous tumor, soft tissue sarcoma, and spindle cell sarcoma), malignant bone tumor, bladder cancer, acute myeloid leukemia, myelodysplastic syndrome, and other hematologic cancers or precancerous lesions. Here, the precancerous lesion refers to, in the narrow sense, a lesion formed by the proliferation of cells at an intermediate stage of carcinogenesis, but in the broad sense, in addition to such lesions, includes lesions prone to carcinogenesis (carcinogenesis-prone states) and lesions that do not themselves lead to carcinogenesis but serve as markers of carcinogenesis-prone states. The pharmaceutical composition of the present invention can be used as a drug for a preventive or therapeutic agent for these cancers or precancerous lesions, a cancer growth inhibitor, a cancer metastasis suppressor, a therapeutic agent such as an apoptosis-promoting agent, and the like.

In particular, the pharmaceutical composition of the present invention can be used as a drug for the treatment of myelodysplastic syndrome, acute myeloid leukemia, or ovarian cancer.

In a particular embodiment of the present invention, the pharmaceutical composition of the present invention may be a combination agent for preventing or treating myelodysplastic syndrome, acute myeloid leukemia, or a precancerous lesion thereof, the composition including, in combination, a first ingredient that is a CLK inhibitor containing Compound A or a salt or a hydrate thereof, and a second ingredient containing at least one antitumor agent selected from the group consisting of quizartinib, midostaurin, cytarabine, venetoclax, and azacitidine.

Further, in a particular embodiment of the present invention, the pharmaceutical composition of the present invention may be a combination agent for preventing or treating ovarian cancer or a precancerous lesion thereof, the composition including, in combination, a first ingredient that is a CLK inhibitor containing Compound A or a salt or a hydrate thereof, and a second ingredient containing at least one antitumor agent selected from the group consisting of niraparib, docetaxel, carboplatin, and paclitaxel.

### (4) Mode of Administration

The pharmaceutical composition of the present invention may be any composition as long as the CLK inhibitor containing the Compound A or a derivative thereof and the antitumor agent can be combined at the time of administration. Accordingly, the pharmaceutical composition of the present invention may be a single formulation (for example, a combination drug) obtained by simultaneously formulating the CLK inhibitor containing the Compound A or a derivative thereof and the at least one antitumor agent, or may be a combination of at least two formulations obtained by separately formulating the CLK inhibitor containing the Compound A or a derivative thereof and the at least one antitumor agent.

Accordingly, the mode of administration is not particularly limited, and examples thereof include (i) administration as a composition containing the CLK inhibitor containing the Compound A or a derivative thereof and the at least one antitumor agent, that is, administration as a single formulation; (ii) simultaneous administration via the same administration route of at least two formulations obtained by separately formulating the CLK inhibitor containing the Compound A or a derivative thereof and the at least one antitumor agent; (iii) administration at different times via the same administration route of at least two formulations obtained by separately formulating the CLK inhibitor containing the Compound A or a derivative thereof and the at least one antitumor agent (for example, administration in the order of the CLK inhibitor containing the Compound A or a derivative thereof followed by the antitumor agent, or in the reverse order); (iv) simultaneous administration via different administration routes of at least two formulations obtained by separately formulating the CLK inhibitor containing the Compound A or a derivative thereof and the at least one antitumor agent; and (v) administration at different times via different administration routes of at least two formulations obtained by separately formulating the CLK inhibitor containing the Compound A or a derivative thereof and the at least one antitumor agent (for example, administration in the order of the CLK inhibitor containing the Compound A or a derivative thereof and the antitumor agent, or in the reverse order).

When the pharmaceutical composition of the present invention is a combination of at least two formulations obtained by separately formulating the CLK inhibitor containing the Compound A or a derivative thereof and the at least one antitumor agent, these formulations may be administered simultaneously or sequentially. Here, the sequential administration includes administration of at least two formulations at different times.

The pharmaceutical composition of the present invention is used as a drug, and may be used, either as a single formulation containing as active ingredients the CLK inhibitor containing the Compound A or a derivative thereof and the at least one antitumor agent, or as at least two formulations obtained by separately formulating them, in the form of solid, semi-solid, or liquid pharmaceutical preparations suitable for rectal, nasal, pulmonary, vaginal, topical (local), oral, or parenteral (including subcutaneous, implantation, intravenous, and intramuscular) administration, such as tablets, pellets, troches, capsules, suppositories, creams, ointments, aerosols, powders, solutions, emulsions, suspensions, syrups, and injections.

The pharmaceutical composition of the present invention is stable, has low toxicity, and can be used safely. The dose of the Compound A used in the combination of the pharmaceutical compositions of the present invention varies depending on the condition, the body weight, and the age of the patient, the type of the compound, the administration method, the administration route, and the like, but is adjusted so as to bring about the desired effect. For example, the daily dose of the Compound A used in the combination of the pharmaceutical compositions of the present invention, when orally administered, is usually from about 0.01 mg to about 20 mg, preferably from about 0.01 mg to about 10 mg, more preferably from about 0.01 mg to about 5 mg, per kg of body weight, and these may be administered once or divided into multiple administrations such as two or three times. The dose of the antitumor agent used in the combination of the pharmaceutical compositions of the present invention also varies depending on the condition, the body weight, and the age of the patient, the type of the compound, the administration method, the administration route, and the like, but is adjusted so as to bring about the desired effect. In the combination of the pharmaceutical compositions of the present invention, the blending ratio between the Compound A and the antitumor agent may be appropriately selected depending on the administration subject, the administration route, the target disease, the symptoms, the specific combination of drugs, and the like. For example, when the administration subject is a human subject, from 0.01 parts to 100 parts by weight of the antitumor agent may be used per 1 part by weight of the Compound A.

It should be noted that, in the disclosure of the present specification, the term "effective amount" means an amount that, when a compound or a combination of one or more compounds is administered simultaneously or sequentially, induces the desired biological response or pharmacological effect.

Further, the pharmaceutical composition of the present invention may include a commercial package which includes a written matter describing the dose and the administration method such that the CLK inhibitor containing the Compound A or a derivative thereof and the antitumor agent are used in combination or administered in combination.

In the present specification, specific matters described in one embodiment according to each aspect of the present invention may be arbitrarily combined to form a new embodiment, and such a new embodiment should also be understood as being included in each aspect of the present invention.

### Examples

Hereinafter, the present invention will be described more specifically by way of Examples and the like, but the present invention is not to be limited in any way by these Examples and the like.

### Example 1: Combined effect of the Compound A and the antitumor agent

In order to determine whether there is a synergistic effect or an additive effect between the Compound A and the antitumor agent in the combination of the pharmaceutical compositions of the present invention, a dose-response curve for each compound alone was first determined, and further, the cell growth inhibitory effect was measured when the Compound A and the antitumor agent were mixed at three fixed ratios of 50% : 50% (1:1), 80% : 20% (4:1), and 20% : 80% (1:4) of the IC50 value for the evaluation-target cells of the compound, respectively. The presence or the absence of a synergistic effect or an additive effect on the cell growth inhibition as an evaluation index was determined by curve-shift analysis, and the degree thereof was subsequently quantified by CI (Combination Index).

All cell-line stocks used in this assay were all subcultured in ATCC-recommended medium and were prepared by a freezing method according to the ATCC-recommended protocol.

### (i) Preparation of single formulation and mixture solution of the compound

The Compound A and the antitumor agent were each stored in powder form under conditions recommended by the supplier, and thereafter the powder weighed on a calibrated balance was dissolved in 100% DMSO and stored at room temperature. However, Cisplatin, Melphalan, and Oxaliplatin were used after being dissolved in water. The compound stocks thus prepared were further diluted with DMSO on the day of experiment to a concentration 3160-fold the respective IC50, and thereafter mixed at three fixed ratios (1:1, 1:4, and 4:1). It should be noted that, since in these mixture solutions the finally expected growth-inhibitory effect was matched on the basis of the IC50 of each compound, all compound stock solutions were assumed in theory to have equal potency. However, for the antitumor agent group for which the IC50 in single agent treatment was not calculated (IC50 = Not Available), mixing at the above fixed ratio was not carried out, and the Compound A was mixed at 1:1 so that the constant concentration shown in Tables 2 and 4 would be the final concentration. The concentrations of the Compound A before the mixing were, respectively, 10.00-fold, 3.16-fold, 1.00-fold, 0.32-fold, 0.10-fold, 0.03-fold, and 0.01-fold of the IC50.

Next, in the experiments using the human promyelocytic leukemia cell-derived cell line HL-60 and the human lung cancer cell line NCI-H661, seven compound dilutions were prepared by diluting with DMSO at a common ratio of 3 a compound stock in which the Compound A and the antitumor agent at a concentration 3160-fold the IC50 had each been mixed at the above fixed ratio. After further dilution 31.6-fold using sterilized 20 mM Hepes buffer (with a pH value of 7.4), 5 µl of the single formulation or the mixture solution of the compound was added in duplicate (n = 2) to 45 µl of cell culture solution in a 384-well plate assay. The final DMSO concentration after the addition of the single formulation or the mixture solution of the compound was 0.4%. The final assay concentration range in the case of the single formulation of the compound was, in all cases, 10 µM, 3.16 µM, 1.00 µM, 0.32 µM, 0.10 µM, 0.03 µM, and 0.01 µM.

In the case of the human promyelocytic leukemia-derived cell line MV-4-11, the compounds were diluted using a MultiDrop (Thermo) to prepare a compound solution at 10-fold the final concentration. Specifically, the Compound A and the antitumor agent were each mixed at the above fixed ratio, and the concentrations of nine compound dilutions were set by diluting at a common ratio of 2 (only Venetoclax was diluted at a common ratio of 3), with the maximum dose being 160-fold the IC50 (only Venetoclax was 810-fold). Fifty microliters of a cell-culture medium was placed into a 96-well V-bottom plate for compound preparation, and the Compound A and the antitumor agent were added using MultiDrop so as to reach the set concentrations. The DMSO concentration in each well was normalized with DMSO so as to be 1% in all wells. Ten microliters of the single formulation or the mixture solution of the compound was added in triplicate (n = 3) to 90 µl of the cell culture solution in a 96-well plate assay. The final DMSO concentration after the addition of the single formulation or the mixture solution of the compound was 0.1% (0.2% only for Venetoclax). For the single formulation of the compound, the concentrations were set to be the same as those of each compound contained in the mixture solution.

### (ii) Cell proliferation assay

In the experiments using the human promyelocytic leukemia cell-derived cell line HL-60 and the human lung cancer cell line NCI-H661, stocks of the human promyelocytic leukemia cell-derived cell line HL-60 and the human lung cancer cell line NCI-H661 were thawed, diluted with an appropriate medium, dispensed into 384-well plates in 45 µl of the medium at from 200 to 3200 cells depending on the cell line used, and the outer edges of each 384-well plate were filled with phosphate-buffered saline. The seeded cells were cultured for 24 hours at 37°C under humidified conditions with 5% CO2, then 5 µl of the single formulation or the mixture solution of the compound prepared above was added, and the cells were further cultured for 72 hours. However, for EPZ-6438, JQ1, MLN-4924, MLN-7243, MPI-0479605, and NMS-P715, the cells were cultured for 120 hours. After culture, the plate was left at room temperature for 30 minutes, after which 25 µl of the ATPlite 1Step^{™} solution (available from Perkin Elmer) was added to each well and shaken for two minutes. After shaking for ten minutes at room temperature in the dark, luminescence was measured with a multimode reader, Envision (available from Perkin Elmer). In the experiment using the human promyelocytic leukemia-derived cell line MV-4-11, a stock of the human promyelocytic leukemia-derived cell line MV-4-11 was thawed, diluted with an appropriate medium, dispensed into a 96-well plate in 90 µl of the medium at 20,000 cells, and the outer edges of each 96-well plate were filled with phosphate-buffered saline. Ten microliters of the single formulation or the mixture solution of the compound prepared above was added, and the cells were further cultured for 72 hours. After the culture, 100 µl of the CellTiter-Glo^{™} 2.0 Reagent (available from Promega) was added to each well, the plate was shaken for 30 minutes at room temperature in the dark, and luminescence was measured with an EnSight multimode plate reader (available from Perkin Elmer).

### (iii) Measurement at time 0 of culture

In the case of the HL-60 and NCI-H661 cell lines, 45 µl of cells was dispensed with n = 4, 5 µl of the DMSO-containing Hepes buffer and 25 µl of the ATPlite 1Step^{™} solution (available from Perkin Elmer) were added, and the mixture solution was mixed for two minutes. After ten minutes of culture at room temperature in the dark, luminescence was measured. In the case of the MV-4-11 cell line, 90 µl of cells was dispensed with n = X, 100 µl of the CellTiter-Glo^{™} was added to each well, the plate was shaken for 30 minutes at room temperature in the dark, and luminescence was measured with an EnSight multimode plate reader (available from Perkin Elmer).

### (iv) Adjustment of cell proliferation

The time required for cell division of all cell lines was calculated from the growth signal at t = 0 hours and that of untreated cells at t = 72 hours. If the time required for cell division deviated from the past average by from 0.5- to 2.0-fold, the assay was judged invalid.

### (v) Maximum luminescence

In the case of the HL-60 and NCI-H661 cell lines, for each 384-well plate, the maximum luminescence was measured after 72 hours of culture under conditions without compound in the presence of 0.4% DMSO. The value was calculated by averaging n = 14 values. This average value is defined as the luminescence of the untreated group after t = 72 hours. In the case of the MV-4-11 cell line, for each 96-well plate, the maximum luminescence was measured after 72 hours of culture under conditions without compound in the presence of 0.1% DMSO. The value was calculated by averaging n = 3 values. This average value is defined as the luminescence of the untreated group after t = 72 hours.

### (vi) Dose-dependent curve

In this assay, an accurate IC50 value for each compound alone is required. Therefore, the dose-response signal for each compound alone was represented by the following 4-parameter logistic curve using XL-fit5 (IDBS): Luminescence = bottom + (top-bottom) / (1 + 10^{(logIC50-log[cpd])·hill)} (wherein cpd indicates the concentration of the test compound, hill indicates the hill coefficient, and bottom and top indicate the minimum and maximum values of the curve, respectively.)

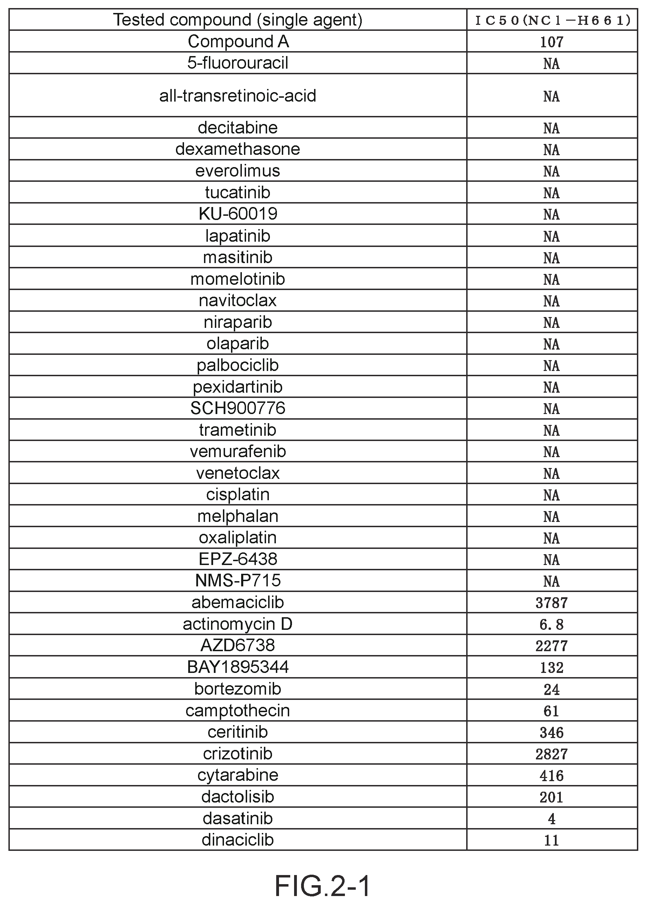

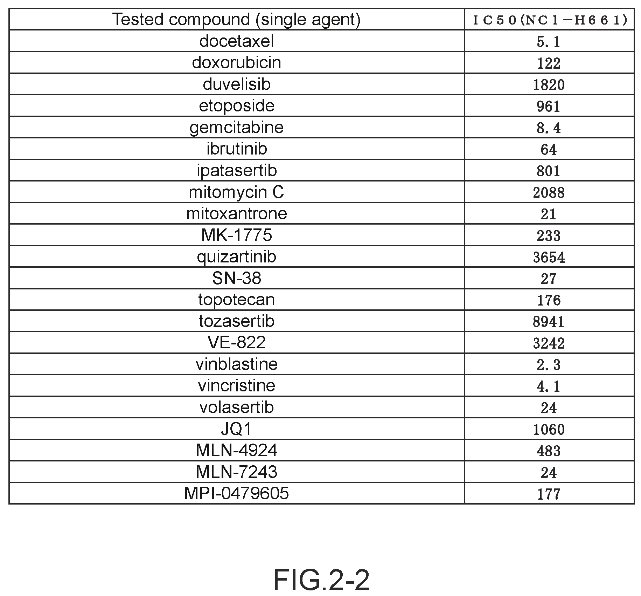

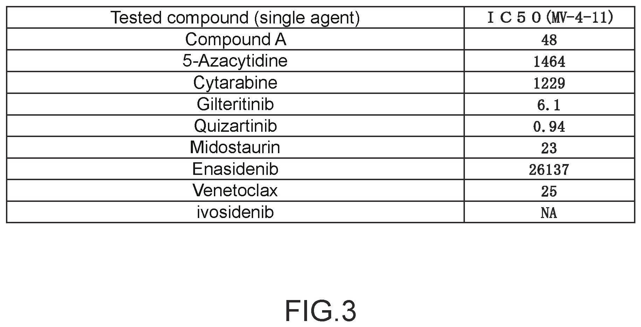

Fig. 1, Fig. 2, and Fig. 3 show the IC50 values of each compound alone for each cell line (HL-60, NCI-H661, and MV-4-11, respectively).

### (vii) Curve-shift analysis

This assay allows visual confirmation of a synergistic effect.

The concentration of the mixture solution of the Compound A (cpd1) and the antitumor agent (cpd2) was calculated from the concentration and IC50 "units" of each compound alone according to the following equation: $\left[mix\right] = \left[cpd1\right] / {IC}_{50 , cpd 1} + \left[cpd2\right] / {IC}_{50 , cpd 2}$ (wherein mix indicates the concentration of the mixture solution, cpd1 indicates the concentration of the Compound A, and cpd2 indicates the concentration of the antitumor agent.)

Further, the dose-response signal of the mixture solution was represented by the following 4-parameter logistic curve using XL-fit5 (IDBS):
Luminescence = bottom + (top-bottom) / (1 + 10^{(logX-log[mix])·hill)}) (wherein hill indicates the hill coefficient, X is the inflection point of the curve, and bottom and top indicate the minimum and maximum values of the curve, respectively.)

Here, since the mixture solution [mix] in the equation is expressed by the IC50 value, when the IC50 values are comparable, the curves for the single formulations of the compound overlap with each other, and their inflection point becomes 1 as a value. Here, the IC50 value used for the calculation is the value determined for the IC50 value of the single formulation of the compound.

For a mixture solution having no synergistic effect, the curve overlaps with the curve of each compound alone. When the mixture solution has a synergistic effect, the curve shifts to the left and the IC50 value decreases. In other words, the mixture solution shows a more powerful cell-growth inhibitory effect than the effect expected on the basis of each compound alone, which serves as a good indicator in the evaluation of a synergistic effect.

For the mixture solution of the Compound A and the antitumor agent, the IC50 curve shifted to the left for any blending ratio from 1:4 to 4:1, and a synergistic effect was observed as compared with the case where each compound was used alone.

### (viii) Determination of CI

CI is one of the most widely used quantitative indices of synergistic effects. CI is used when evaluating the concentration required to achieve a certain "specific effect"; for example, CI = 0.1 indicates that the compound concentration required to show the specific effect is one-tenth of the concentration predicted from the data of the single formulation of the compound to be required to achieve the same level of effect. As another example, a combination with CI = 0.1 (a combination of a potent compound and a weak compound) means that the weak compound improves the effective concentration of the potent compound 10-fold. As described above, CI is defined for a certain specific effect (effect F) (for example, F = 0.75 in the case of a 75% effect).

Accordingly, the concentrations required to reach effect F when the Compound A and the antitumor agent were combined were compared with the concentrations required for each compound alone (designated cpd1 and cpd2, respectively).

CIF was calculated by the following equation; ${CI}_{F} = {\left[cpd1\right]}_{F} / {IC}_{F , cpd 1} + {\left[cpd2\right]}_{F} / {IC}_{F , cpd 2}$

In the equation, when [cpd1]0.75 is used, it means the concentration of the Compound A alone in the mixture solution that produces a 75% inhibitory effect (F = 0.75), and IC0.75, cpd1 indicates the IC75 of the Compound A alone. The range of F values (from 0 to 1.0) was calculated using the Calcusyn^{™} program for the results of the HL-60 and NCI-H661 cell lines, and using the GraphPad Prism9 program for the results of the MV-4-11 cell line. A CI of less than 1 indicates a synergistic effect, and in particular, a CI of less than 0.3 means a strong synergistic effect.

For each combination of the Compound A and the antitumor agent, CI was plotted as a function of F.

The evaluation of CI is as shown in Table 1. An additive effect is recognized when 0.9 ≤ CI ≤ 1.1, and a synergistic effect is recognized when CI < 0.9.

**[Table 1]**

| Cl | Effect |
|---|---|
| <0.1 | A very strong synergistic effect is observed |
| 0.1-0.3 | A strong synergistic effect is observed |
| 0.3-0.7 | A synergistic effect is observed |
| 0.7-0.85 | A weak synergistic effect is observed |
| 0.85-0.90 | A very weak synergistic effect is observed |
| 0.90-1.10 | An additive effect is observed |
| 1.10-1.20 | A very weak antagonistic action is observed |
| 1.20-1.45 | A weak antagonistic action is observed |
| 1.45-3.3 | An antagonistic action is observed |
| 3.3-10 | A strong antagonistic action is observed |
| <10 | A very strong antagonistic action is observed |

Tables 2 and 3 below show the CI values calculated for each combination of the Compound A and the antitumor agent in HL-60 cells.

**[Table 2-1]**

| Combined compound | Fixed concentration (µM) | Fa | Cl |
|---|---|---|---|
| 5-Fluorouracil | 1 | 50 | 1.06 |
| | 1 | 75 | 0.92 |
| | 1 | 90 | 0.78 |
| cyclophosphamide | 3.2 | 50 | 0.96 |
| | 3.2 | 75 | 1.01 |
| | 3.2 | 90 | 1.06 |
| dexamethasone | 3.2 | 50 | 1.03 |
| | 3.2 | 75 | 1.00 |
| | 3.2 | 90 | 1.00 |
| everolimus | 1 | 50 | 0.61 |
| | 1 | 75 | 0.77 |
| | 1 | 90 | 0.93 |
| ibrutinib | 3.2 | 50 | 1.04 |
| imatinib | 3.2 | 50 | 1.01 |
| tucatinib | 3.2 | 50 | 1.03 |
| | 3.2 | 75 | 0.81 |
| | 3.2 | 90 | 0.60 |
| KU-60019 | 1 | 50 | 0.84 |
| | 1 | 75 | 0.82 |
| | 1 | 90 | 0.82 |
| lapatinib | 1 | 50 | 0.97 |
| | 1 | 75 | 1.09 |
| masitinib | 3.2 | 50 | 0.90 |
| momelotinib | 1 | 50 | 0.72 |
| | 1 | 75 | 0.75 |
| | 1 | 90 | 0.75 |
| niraparib | 1 | 50 | 0.75 |
| | 1 | 75 | 0.87 |
| | 1 | 90 | 1.01 |
| olaparib | 3.2 | 50 | 0.72 |
| | 3.2 | 75 | 0.73 |
| | 3.2 | 90 | 0.76 |
| pexidartinib | 3.2 | 50 | 0.78 |
| | 3.2 | 75 | 0.85 |
| | 3.2 | 90 | 0.96 |

**[Table 2-2]**

| Combined compound | Fixed concentration (µM) | Fa | Cl |
|---|---|---|---|
| ruxolitinib | 3.2 | 50 | 0.80 |
| | 3.2 | 75 | 0.93 |
| | 3.2 | 90 | 1.10 |
| UMI-77 | 1 | 50 | 0.88 |
| | 1 | 75 | 0.82 |
| | 1 | 90 | 0.75 |
| vemurafenib | 1 | 75 | 1.02 |
| | 1 | 90 | 0.93 |
| cisplatin | 0.32 | 50 | 0.95 |
| | 0.32 | 75 | 0.97 |
| | 0.32 | 90 | 0.99 |
| melphalan | 3.2 | 50 | 0.79 |
| | 3.2 | 75 | 0.74 |
| | 3.2 | 90 | 0.67 |
| oxaliplatin | 1 | 50 | 0.87 |
| | 1 | 75 | 0.85 |
| | 1 | 90 | 0.83 |
| EPZ-6438 | 0.32 | 50 | 0.86 |
| | 0.32 | 75 | 0.85 |
| | 0.32 | 90 | 0.83 |

**[Table 3-1]**

| Combined compound | Blending ratio (Compound A : combined compound) | Fa | CI |
|---|---|---|---|
| abemaciclib | 1:1 | 50 | 1.01 |
| | 1:1 | 75 | 1.10 |
| | 4:1 | 50 | 0.93 |
| | 4:1 | 75 | 0.91 |
| | 4:1 | 90 | 0.92 |
| | 1:4 | 50 | 0.99 |
| | 1:4 | 75 | 1.03 |
| actinomycin D | 4:1 | 50 | 0.91 |
| | 4:1 | 75 | 1.04 |
| | 1:4 | 50 | 1.04 |
| all-transretinoic-acid | 1:1 | 50 | 0.79 |
| | 1:1 | 75 | 1.10 |
| | 4:1 | 50 | 0.80 |
| | 4:1 | 75 | 1.07 |
| | 1:4 | 50 | 0.61 |
| AZD6738 | 1:1 | 50 | 0.81 |
| | 1:1 | 75 | 0.60 |
| | 1:1 | 90 | 0.47 |
| | 4:1 | 50 | 0.84 |
| | 4:1 | 75 | 0.81 |
| | 4:1 | 90 | 0.79 |
| | 1:4 | 50 | 0.81 |
| | 1:4 | 75 | 0.58 |
| | 1:4 | 90 | 0.43 |
| BAY1895344 | 1:1 | 50 | 0.72 |
| | 1:1 | 75 | 0.58 |
| | 1:1 | 90 | 0.45 |
| | 4:1 | 50 | 0.87 |
| | 4:1 | 75 | 0.71 |
| | 4:1 | 90 | 0.57 |
| | 1:4 | 50 | 0.97 |
| | 1:4 | 75 | 0.63 |
| | 1:4 | 90 | 0.27 |
| bortezomib | 4:1 | 50 | 1.08 |
| camptothecin | 4:1 | 50 | 0.97 |
| ceritinib | 1:1 | 75 | 1.04 |
| | 1:1 | 90 | 0.75 |
| | 4:1 | 90 | 1.03 |
| | 1:4 | 75 | 0.98 |
| | 1:4 | 90 | 0.68 |
| crizotinib | 4:1 | 90 | 1.05 |
| | 1:4 | 90 | 1.09 |

**[Table 3-2]**

| Combined compound | Blending ratio (Compound A : combined compound) | Fa | CI |
|---|---|---|---|
| cytarabine | 4:1 | 75 | 1.09 |
| | 4:1 | 90 | 1.03 |
| dactolisib | 1:1 | 75 | 0.86 |
| | 1:1 | 90 | 0.58 |
| | 4:1 | 75 | 1.09 |
| | 4:1 | 90 | 1.02 |
| | 1:4 | 75 | 0.95 |
| | 1:4 | 90 | 0.43 |
| dasatinib | 1:1 | 50 | 0.95 |
| | 1:1 | 75 | 0.69 |
| | 1:1 | 90 | 0.50 |
| | 4:1 | 50 | 1.09 |
| | 4:1 | 75 | 0.80 |
| | 4:1 | 90 | 0.58 |
| | 1:4 | 75 | 0.97 |
| | 1:4 | 90 | 0.81 |
| decitabine | 1:1 | 50 | 0.65 |
| | 1:1 | 75 | 0.90 |
| | 4:1 | 50 | 0.85 |
| | 4:1 | 75 | 0.83 |
| | 4:1 | 90 | 0.84 |
| | 1:4 | 50 | 0.48 |
| | 1:4 | 75 | 0.59 |
| | 1:4 | 90 | 1.10 |
| docetaxel | 1:1 | 90 | 0.87 |
| | 4:1 | 75 | 1.05 |
| | 4:1 | 90 | 0.91 |
| | 1:4 | 75 | 1.10 |
| doxorubicin | 1:1 | 90 | 1.02 |
| | 4:1 | 75 | 1.02 |
| | 4:1 | 90 | 0.84 |
| | 1:4 | 75 | 1.01 |
| | 1:4 | 90 | 0.83 |
| duvelisib | 1:1 | 50 | 0.75 |
| | 1:1 | 75 | 0.95 |
| | 4:1 | 50 | 0.66 |
| | 4:1 | 75 | 0.91 |
| | 1:4 | 50 | 0.47 |
| | 1:4 | 75 | 0.75 |

**[Table 3-3]**

| Combined compound | Blending ratio (Compound A : combined compound) | Fa | CI |
|---|---|---|---|
| entinostat | 1:1 | 75 | 1.04 |
| | 1:1 | 90 | 0.78 |
| | 4:1 | 75 | 0.92 |
| | 4:1 | 90 | 0.70 |
| epirubicin | 4:1 | 50 | 1.10 |
| | 1:4 | 50 | 1.07 |
| etoposide | 1:1 | 50 | 0.87 |
| | 1:1 | 75 | 0.95 |
| | 1:1 | 90 | 1.02 |
| | 4:1 | 50 | 0.77 |
| | 4:1 | 75 | 0.82 |
| | 4:1 | 90 | 0.88 |
| | 1:4 | 50 | 0.77 |
| | 1:4 | 75 | 0.67 |
| | 1:4 | 90 | 0.61 |
| ipatasertib | 1:1 | 50 | 0.76 |
| | 1:1 | 75 | 0.74 |
| | 1:1 | 90 | 0.79 |
| | 4:1 | 50 | 0.79 |
| | 4:1 | 75 | 0.86 |
| | 4:1 | 90 | 0.96 |
| | 1:4 | 50 | 0.71 |
| | 1:4 | 75 | 0.57 |
| | 1:4 | 90 | 0.71 |
| mercaptopurine | 1:1 | 75 | 1.02 |
| | 1:1 | 90 | 0.90 |
| | 4:1 | 50 | 1.00 |
| | 4:1 | 75 | 1.01 |
| | 4:1 | 90 | 1.06 |
| methotrexate | 4:1 | 50 | 1.03 |
| | 4:1 | 75 | 1.00 |
| | 4:1 | 90 | 0.93 |
| | 1:4 | 90 | 0.70 |
| mitomycin C | 1:1 | 90 | 0.96 |
| | 4:1 | 50 | 1.06 |
| | 4:1 | 75 | 1.00 |
| | 4:1 | 90 | 0.93 |
| | 1:4 | 75 | 1.08 |
| | 1:4 | 90 | 0.97 |
| mitoxantrone | 4:1 | 50 | 0.85 |
| | 4:1 | 75 | 0.93 |
| | 4:1 | 90 | 1.04 |

**[Table 3-4]**

| Combined compound | Blending ratio (Compound A : combined compound) | Fa | CI |
|---|---|---|---|
| MK-1775 | 1:1 | 50 | 0.67 |
| | 1:1 | 75 | 0.68 |
| | 1:1 | 90 | 0.77 |
| | 4:1 | 50 | 0.79 |
| | 4:1 | 75 | 0.81 |
| | 4:1 | 90 | 0.89 |
| | 1:4 | 50 | 0.80 |
| | 1:4 | 75 | 0.74 |
| | 1:4 | 90 | 0.62 |
| navitoclax | 1:1 | 50 | 1.05 |
| | 1:1 | 75 | 0.67 |
| | 1:1 | 90 | 0.60 |
| | 4:1 | 50 | 1.10 |
| | 4:1 | 75 | 0.91 |
| | 4:1 | 90 | 0.79 |
| | 1:4 | 50 | 1.06 |
| | 1:4 | 75 | 0.52 |
| | 1:4 | 90 | 0.67 |
| palbociclib | 1:1 | 50 | 1.00 |
| | 4:1 | 50 | 0.89 |
| | 4:1 | 75 | 0.90 |
| | 4:1 | 90 | 0.92 |
| | 1:4 | 50 | 0.79 |
| | 1:4 | 75 | 1.06 |
| SCH900776 | 1:1 | 50 | 0.60 |
| | 1:1 | 75 | 0.66 |
| | 1:1 | 90 | 0.83 |
| | 4:1 | 50 | 0.57 |
| | 4:1 | 75 | 0.63 |
| | 4:1 | 90 | 0.77 |
| | 1:4 | 50 | 0.70 |
| | 1:4 | 75 | 0.90 |
| SN-38 | 4:1 | 50 | 0.99 |
| | 4:1 | 75 | 0.91 |
| | 4:1 | 90 | 0.82 |
| | 1:4 | 90 | 1.07 |

**[Table 3-5]**

| Combined compound | Blending ratio (Compound A : combined compound) | Fa | CI |
|---|---|---|---|
| topotecan | 1:1 | 50 | 0.82 |
| | 1:1 | 75 | 0.87 |
| | 1:1 | 90 | 0.92 |
| | 4:1 | 50 | 0.87 |
| | 4:1 | 75 | 0.91 |
| | 4:1 | 90 | 0.95 |
| | 1:4 | 50 | 0.97 |
| | 1:4 | 75 | 0.96 |
| | 1:4 | 90 | 0.93 |
| tozasertib | 1:1 | 50 | 1.04 |
| | 1:1 | 75 | 1.06 |
| | 1:1 | 90 | 1.09 |
| | 4:1 | 50 | 0.95 |
| | 4:1 | 75 | 0.98 |
| | 4:1 | 90 | 1.00 |
| | 1:4 | 50 | 0.71 |
| | 1:4 | 75 | 0.78 |
| trametinib | 1:1 | 50 | 1.07 |
| | 1:1 | 75 | 0.90 |
| | 1:1 | 90 | 1.01 |
| | 1:4 | 75 | 0.91 |
| VE-822 | 1:1 | 50 | 0.93 |
| | 1:1 | 75 | 0.83 |
| | 1:1 | 90 | 0.76 |
| | 4:1 | 50 | 0.93 |
| | 4:1 | 75 | 0.89 |
| | 4:1 | 90 | 0.85 |
| | 1:4 | 50 | 0.91 |
| | 1:4 | 75 | 1.02 |
| venetoclax | 1:1 | 50 | 0.73 |
| | 1:1 | 75 | 0.71 |
| | 1:1 | 90 | 0.71 |
| | 4:1 | 50 | 0.95 |
| | 4:1 | 75 | 0.82 |
| | 4:1 | 90 | 0.69 |
| | 1:4 | 50 | 0.64 |
| | 1:4 | 75 | 0.65 |
| | 1:4 | 90 | 0.84 |

**[Table 3-6]**

| Combined compound | Blending ratio (Compound A : combined compound) | Fa | CI |
|---|---|---|---|
| vinblastine | 1:1 | 50 | 1.00 |
| | 1:1 | 75 | 0.95 |
| | 1:1 | 90 | 0.81 |
| | 4:1 | 50 | 1.03 |
| | 4:1 | 75 | 1.01 |
| | 4:1 | 90 | 0.91 |
| | 1:4 | 50 | 0.95 |
| | 1:4 | 75 | 0.86 |
| | 1:4 | 90 | 0.52 |
| vincristine | 1:1 | 50 | 0.97 |
| | 1:1 | 75 | 0.75 |
| | 1:1 | 90 | 0.60 |
| | 4:1 | 50 | 1.05 |
| | 4:1 | 75 | 0.85 |
| | 4:1 | 90 | 0.65 |
| | 1:4 | 50 | 1.10 |
| | 1:4 | 75 | 1.04 |
| | 1:4 | 90 | 1.08 |
| volasertib | 1:1 | 50 | 0.99 |
| | 1:1 | 75 | 0.92 |
| | 1:1 | 90 | 0.96 |
| | 4:1 | 50 | 0.97 |
| | 4:1 | 75 | 0.99 |
| | 4:1 | 90 | 1.02 |
| | 1:4 | 50 | 0.90 |
| | 1:4 | 75 | 0.86 |
| | 1:4 | 90 | 0.71 |
| quizartinib | 4:1 | 75 | 0.99 |
| | 4:1 | 90 | 0.84 |
| JQ1 | 1:1 | 75 | 1.09 |
| | 1:1 | 90 | 1.05 |
| | 4:1 | 75 | 1.02 |
| | 4:1 | 90 | 0.98 |
| | 1:4 | 75 | 1.01 |
| | 1:4 | 90 | 0.86 |
| MLN-4924 | 4:1 | 90 | 1.02 |
| MLN-7243 | 4:1 | 50 | 1.07 |
| MPI-0479605 | 1:1 | 90 | 1.10 |
| | 1:4 | 90 | 0.93 |
| NMS-P715 | 4:1 | 90 | 1.07 |

Further, the results for the NCI-H661 cells are also shown in Tables 4 and 5 below.

**[Table 4]**

| Combined compound | Fixed concentration (µM) | Fa | Cl |
|---|---|---|---|
| 5-fluorouracil | 3.2 | 50 | 0.83 |
| all-transretinoic-acid | 1 | 50 | 1.00 |
| | 1 | 75 | 1.05 |
| decitabine | 3.2 | 50 | 1.04 |
| | 3.2 | 75 | 0.10 |
| dexamethasone | 3.2 | 50 | 0.84 |
| everolimus | 0.1 | 50 | 0.78 |
| ibrutinib | 3.2 | 50 | 0.92 |
| KU-60019 | 1 | 50 | 0.81 |
| lapatinib | 1 | 50 | 0.93 |
| | 1 | 75 | 0.76 |
| masitinib | 3.2 | 50 | 0.90 |
| momelotinib | 1 | 50 | 0.93 |
| navitoclax | 3.2 | 50 | 0.88 |
| | 3.2 | 75 | 0.81 |
| | 3.2 | 90 | 0.10 |
| niraparib | 1 | 50 | 0.96 |
| olaparib | 3.2 | 50 | 1.02 |
| | 3.2 | 75 | 0.10 |
| palbociclib | 3.2 | 50 | 0.71 |
| pexidartinib | 3.2 | 50 | 0.69 |
| | 3.2 | 75 | 0.17 |
| SCH900776 | 3.2 | 50 | 0.91 |
| trametinib | 0.32 | 50 | 0.80 |
| vemurafenib | 3.2 | 50 | 0.95 |
| venetoclax | 3.2 | 50 | 0.88 |
| | 3.2 | 75 | 0.52 |
| cisplatin | 0.32 | 50 | 0.92 |
| | 0.32 | 75 0.10 | |
| melphalan | 3.2 | 50 | 0.86 |
| | 3.2 | 75 | 0.77 |
| oxaliplatin | 1 | 50 | 0.77 |
| | 1 | 75 | 0.10 |
| EPZ-6438 | 2 | 50 | 0.77 |
| | 2 | 75 | 0.77 |
| | 2 | 90 | 0.75 |
| NMS-P715 | 1 | 50 | 0.91 |
| | 1 | 75 | 0.93 |
| | 1 | 90 | 0.92 |

**[Table 5-1]**

| Combined compound | Blending ratio (Compound A : combined compound) | Fa | CI |
|---|---|---|---|
| abemaciclib | 1:1 | 75 | 1.02 |
| | 1:1 | 90 | 0.59 |
| | 4:1 | 90 | 0.54 |
| | 1:4 | 50 | 0.98 |
| | 1:4 | 75 | 0.81 |
| | 1:4 | 90 | 0.65 |
| actinomycin D | 1:1 | 50 | 0.94 |
| | 1:1 | 75 | 0.68 |
| | 1:1 | 90 | 0.60 |
| | 4:1 | 50 | 0.93 |
| | 4:1 | 75 | 0.24 |
| | 4:1 | 90 | 0.17 |
| | 1:4 | 50 | 0.94 |
| | 1:4 | 75 | 0.84 |
| | 1:4 | 90 | 0.78 |
| AZD6738 | 1:1 | 50 | 0.78 |
| | 1:1 | 75 | 0.10 |
| | 4:1 | 50 | 0.87 |
| | 4:1 | 75 | 0.10 |
| | 1:4 | 50 | 0.79 |
| | 1:4 | 75 | 0.10 |
| BAY1895344 | 1:1 | 75 | 0.10 |
| | 1:4 | 75 | 0.10 |
| bortezomib | 1:1 | 50 | 1.06 |
| | 1:1 | 75 | 0.90 |
| | 4:1 | 75 | 1.04 |
| | 1:4 | 50 | 1.07 |
| | 1:4 | 75 | 0.97 |
| camptothecin | 1:1 | 50 | 1.04 |
| | 1:1 | 75 | 0.73 |
| | 4:1 | 50 | 1.01 |
| | 4:1 | 75 | 0.77 |
| | 1:4 | 50 | 0.97 |
| | 1:4 | 75 | 0.77 |
| | 1:4 | 90 | 0.10 |
| ceritinib | 1:1 | 50 | 0.64 |
| | 1:1 | 75 | 0.56 |
| | 4:1 | 50 | 0.79 |
| | 4:1 | 75 | 0.50 |
| | 1:4 | 50 | 0.56 |
| | 1:4 | 75 | 0.38 |
| | 1:4 | 90 | 0.10 |

**[Table 5-2]**

| Combined compound | Blending ratio (Compound A : combined compound) | Fa | CI |
|---|---|---|---|
| crizotinib | 1:1 | 75 | 0.81 |
| | 1:1 | 90 | 0.54 |
| | 4:1 | 50 | 1.08 |
| | 4:1 | 75 | 0.54 |
| | 4:1 | 90 | 0.19 |
| | 1:4 | 90 | 1.04 |
| cytarabine | 1:1 | 50 | 1.01 |
| | 4:1 | 50 | 0.96 |
| | 4:1 | 75 | 1.09 |
| | 1:4 | 50 | 0.92 |
| dactolisib | 1:1 | 50 | 0.88 |
| | 1:1 | 75 | 0.72 |
| | 4:1 | 50 | 0.91 |
| | 4:1 | 75 | 0.72 |
| | 1:4 | 50 | 1.06 |
| | 1:4 | 75 | 0.55 |
| dasatinib | 1:1 | 50 | 0.69 |
| | 1:1 | 75 | 1.06 |
| | 4:1 | 50 | 0.80 |
| | 4:1 | 75 | 0.68 |
| | 1:4 | 50 | 0.63 |
| dinaciclib | 1:1 | 50 | 1.04 |
| | 1:1 | 75 | 0.57 |
| | 4:1 | 75 | 0.42 |
| | 1:4 | 90 | 1.04 |
| docetaxel | 1:1 | 50 | 1.06 |
| | 4:1 | 50 | 1.02 |
| | 4:1 | 75 | 0.10 |
| | 1:4 | 75 | 0.10 |
| doxorubicin | 1:1 | 50 | 0.94 |
| | 1:1 | 75 | 0.55 |
| | 1:1 | 90 | 0.66 |
| | 4:1 | 75 | 0.23 |
| | 4:1 | 90 | 0.23 |
| | 1:4 | 50 | 0.98 |
| | 1:4 | 75 | 0.90 |
| | 1:4 | 90 | 1.01 |

**[Table 5-3]**

| Combined compound | Blending ratio (Compound A : combined compound) | Fa | CI |
|---|---|---|---|
| duvelisib | 1:1 | 50 | 0.64 |
| | 1:1 | 75 | 0.37 |
| | 4:1 | 50 | 0.81 |
| | 4:1 | 75 | 0.36 |
| | 1:4 | 50 | 0.77 |
| | 1:4 | 75 | 0.35 |
| entinostat | 1:1 | 50 | 0.95 |
| | 1:1 | 75 | 0.59 |
| | 1:1 | 90 | 0.56 |
| | 4:1 | 50 | 0.85 |
| | 4:1 | 75 | 0.28 |
| | 1:4 | 75 | 0.89 |
| | 1:4 | 90 | 0.67 |
| epirubicin | 1:1 | 50 | 1.03 |
| | 1:1 | 75 | 0.48 |
| | 1:1 | 90 | 0.60 |
| | 4:1 | 75 | 0.16 |
| | 1:4 | 75 | 0.80 |
| | 1:4 | 90 | 0.61 |
| etoposide | 1:1 | 50 | 0.70 |
| | 1:1 | 75 | 0.10 |
| | 4:1 | 50 | 0.85 |
| | 4:1 | 75 | 0.10 |
| | 1:4 | 50 | 0.50 |
| | 1:4 | 75 | 0.10 |
| gemcitabine | 1:1 | 50 | 0.78 |
| | 1:1 | 75 | 0.10 |
| | 4:1 | 50 | 0.97 |
| | 4:1 | 75 | 0.10 |
| | 1:4 | 50 | 0.43 |
| | 1:4 | 75 | 0.10 |
| ibrutinib | 1:1 | 50 | 0.83 |
| | 1:1 | 75 | 0.10 |
| | 4:1 | 50 | 0.95 |
| | 4:1 | 75 | 0.10 |
| | 1:4 | 50 | 0.86 |
| | 1:4 | 75 | 0.10 |

**[Table 5-4]**

| Combined compound | Blending ratio (Compound A : combined compound) | Fa | CI |
|---|---|---|---|
| ipatasertib | 1:1 | 50 | 0.56 |
| | 1:1 | 75 | 0.10 |
| | 4:1 | 50 | 0.78 |
| | 4:1 | 75 | 0.10 |
| | 1:4 | 50 | 0.58 |
| | 1:4 | 75 | 0.10 |
| | 1:4 | 90 | 0.10 |
| mitomycin C | 1:1 | 50 | 0.90 |
| | 1:1 | 75 | 0.89 |
| | 1:1 | 90 | 0.36 |
| | 4:1 | 50 | 1.08 |
| | 4:1 | 75 | 0.73 |
| | 4:1 | 90 | 0.07 |
| | 1:4 | 50 | 1.07 |
| | 1:4 | 75 | 0.84 |
| | 1:4 | 90 | 0.48 |
| mitoxantrone | 1:1 | 50 | 0.79 |
| | 1:1 | 75 | 0.69 |
| | 1:1 | 90 | 0.10 |
| | 4:1 | 50 | 0.79 |
| | 4:1 | 75 | 0.54 |
| | 1:4 | 50 | 0.41 |
| MK-1775 | 1:1 | 75 | 0.86 |
| | 4:1 | 50 | 1.08 |
| | 4:1 | 75 | 0.93 |
| | 1:4 | 50 | 1.03 |
| | 1:4 | 75 | 0.39 |
| quizartinib | 1:1 | 50 | 0.93 |
| | 1:1 | 75 | 0.85 |
| | 4:1 | 50 | 0.81 |
| | 4:1 | 75 | 0.50 |
| | 1:4 | 50 | 1.06 |
| | 1:4 | 75 | 0.78 |
| | 1:4 | 90 | 0.43 |
| SN-38 | 1:1 | 50 | 1.04 |
| | 1:1 | 75 | 0.61 |
| | 1:1 | 90 | 0.10 |
| | 4:1 | 50 | 1.07 |
| | 4:1 | 75 | 0.43 |
| | 1:4 | 50 | 0.77 |

**[Table 5-5]**

| Combined compound | Blending ratio (Compound A : combined compound) | Fa | CI |
|---|---|---|---|
| topotecan | 1:1 | 50 | 0.88 |
| | 1:1 | 75 | 0.65 |
| | 1:1 | 90 | 0.10 |
| | 4:1 | 50 | 1.07 |
| | 4:1 | 75 | 0.56 |
| | 1:4 | 75 | 0.65 |
| | 1:4 | 90 | 0.10 |
| tozasertib | 1:1 | 50 | 0.94 |
| | 1:1 | 75 | 0.69 |
| | 1:1 | 90 | 0.57 |
| | 4:1 | 75 | 0.63 |
| | 1:4 | 50 | 0.99 |
| | 1:4 | 75 | 0.80 |
| | 1:4 | 90 | 0.57 |
| VE-822 | 1:1 | 50 | 0.89 |
| | 1:1 | 75 | 0.94 |
| | 1:1 | 90 | 0.63 |
| | 4:1 | 90 | 0.56 |
| | 1:4 | 50 | 0.70 |
| | 1:4 | 75 | 0.99 |
| vinblastine | 1:1 | 50 | 1.01 |
| | 1:1 | 75 | 0.54 |
| | 4:1 | 50 | 0.90 |
| | 4:1 | 75 | 0.56 |
| | 1:4 | 50 | 0.75 |
| vincristine | 1:1 | 50 | 0.74 |
| | 1:1 | 75 | 0.45 |
| | 4:1 | 50 | 0.79 |
| | 4:1 | 75 | 0.56 |
| | 1:4 | 50 | 0.89 |
| | 1:4 | 75 | 0.25 |
| volasertib | 1:1 | 75 | 0.10 |
| | 4:1 | 50 | 1.05 |
| | 4:1 | 75 | 0.10 |
| | 1:4 | 50 | 0.79 |

**[Table 5-6]**

| Combined compound | Blending ratio (Compound A : combined compound) | Fa | CI |
|---|---|---|---|
| JQ1 | 1:1 | 50 | 0.66 |
| | 1:1 | 75 | 0.44 |
| | 1:1 | 90 | 0.43 |
| | 4:1 | 50 | 0.78 |
| | 4:1 | 75 | 0.69 |
| | 4:1 | 90 | 0.67 |
| | 1:4 | 50 | 0.65 |
| | 1:4 | 75 | 0.35 |
| | 1:4 | 90 | 0.58 |
| MLN-4924 | 1:1 | 50 | 0.76 |
| | 1:1 | 75 | 0.35 |
| | 1:1 | 90 | 0.31 |
| | 4:1 | 50 | 0.91 |
| | 4:1 | 75 | 0.63 |
| | 4:1 | 90 | 0.55 |
| | 1:4 | 50 | 0.88 |
| | 1:4 | 75 | 0.30 |
| | 1:4 | 90 | 0.43 |
| MLN-7243 | 1:1 | 90 | 1.06 |
| | 4:1 | 50 | 1.03 |
| | 4:1 | 75 | 0.90 |
| | 4:1 | 90 | 0.77 |
| | 1:4 | 90 | 1.05 |
| MPI-0479605 | 1:1 | 50 | 1.04 |
| | 1:1 | 75 | 1.03 |
| | 1:1 | 90 | 1.00 |
| | 4:1 | 50 | 0.87 |
| | 4:1 | 75 | 0.88 |
| | 4:1 | 90 | 0.91 |
| | 1:4 | 50 | 0.65 |
| | 1:4 | 75 | 0.80 |
| | 1:4 | 90 | 0.93 |

Further, the results for the MV-4-11 cells are shown in Table 6 below.

**[Table 6-1]**

| Combined compound | Blending ratio (Compound A : combined compound) | Fa | CI |
|---|---|---|---|
| 5-Azacytidine | 4:1 | 50 | 0.91 |
| | 4:1 | 60 | 0.94 |
| | 4:1 | 70 | 0.98 |
| | 1:4 | 50 | 0.87 |
| | 1:4 | 60 | 0.93 |
| | 1:4 | 70 | 0.99 |
| Cytarabine | 1:1 | 50 | 0.99 |
| | 1:1 | 75 | 0.98 |
| | 1:1 | 90 | 1.01 |
| | 4:1 | 50 | 1.05 |
| | 4:1 | 75 | 0.96 |
| | 4:1 | 90 | 0.91 |
| Gilteritinib | 4:1 | 50 | 0.90 |
| | 4:1 | 75 | 0.99 |
| | 4:1 | 90 | 1.09 |
| | 1:4 | 50 | 0.85 |
| | 1:4 | 75 | 0.93 |
| | 1:4 | 90 | 0.97 |
| Quizartinib | 1:1 | 50 | 1.02 |
| | 1:1 | 75 | 1.04 |
| | 1:1 | 90 | 0.94 |
| | 4:1 | 50 | 1.00 |
| | 4:1 | 75 | 1.03 |
| | 4:1 | 90 | 1.02 |
| | 1:4 | 50 | 0.87 |
| | 1:4 | 75 | 0.96 |
| | 1:4 | 90 | 0.84 |
| Midostaurin | 1:1 | 50 | 0.90 |
| | 1:1 | 75 | 1.00 |
| | 4:1 | 50 | 0.90 |
| | 4:1 | 75 | 0.89 |
| | 4:1 | 90 | 0.92 |
| | 1:4 | 50 | 0.92 |
| | 1:4 | 75 | 1.00 |
| | 1:4 | 90 | 1.06 |
| Enasidenib | 4:1 | 75 | 0.97 |
| | 4:1 | 90 | 0.86 |
| | 1:4 | 50 | 1.10 |
| Venetoclax | 1:1 | 50 | 0.80 |
| | 1:1 | 75 | 0.73 |
| | 1:1 | 90 | 0.73 |
| | 4:1 | 50 | 0.88 |
| | 4:1 | 75 | 0.88 |
| | 4:1 | 90 | 0.92 |
| | 1:4 | 50 | 0.78 |
| | 1:4 | 75 | 0.66 |
| | 1:4 | 90 | 0.60 |

**[Table 6-2]**

| Combined compound | Fixed concentration (µM) | Fa | CI |
|---|---|---|---|
| | 10 | 50 | 0.87 |
| | 10 | 75 | 0.89 |
| | 10 | 90 | 0.90 |
| | 3 | 50 | 0.97 |
| | 3 | 75 | 0.96 |
| | 3 | 90 | 0.95 |
| | 1 | 50 | 0.94 |
| | 1 | 75 | 0.94 |
| | 1 | 90 | 0.94 |
| | 0.3 | 50 | 0.97 |
| | 0.3 | 75 | 0.94 |
| | 0.3 | 90 | 0.92 |
| | 0.1 | 50 | 0.95 |
| | 0.1 | 75 | 0.92 |
| ivosidenib | 0.1 | 90 | 0.91 |

As is apparent from the above results, in both hematologic tumors and solid tumors, the cell proliferation inhibitory effect produced by the combination of the Compound A and the antitumor agent was recognized to be remarkable as compared with the case where the Compound A or the antitumor agent was used alone.

### Example 2: Combined effect of the Compound A and an anti-PD-1 antibody

In order to determine whether the combination of the pharmaceutical compositions of the present invention exhibited an additive effect or a synergistic effect in the antitumor effect, the Compound A and an anti-mouse PD-1 antibody as the antitumor agent were combined and administered in combination to mice subcutaneously bearing a tumor. The amount of change in tumor volume of the subcutaneously transplanted tumor during the administration period was compared between a control group and a group with the administration of a drug and evaluated using the effect indicator T/C. For the determination of the combined effect, the presence or the absence of an additive effect or a synergistic effect was statistically analyzed by two-way analysis of variance using the amount of change in the tumor volume of each individual. MC38 cells (mouse colorectal cancer cell line) and female C57BL/6 mice with the age of from seven to nine weeks were prepared by the contract research organization Crown Bioscience, Inc.

### (i) Preparation of drug solutions to be administered

The Compound A was prepared as a suspension at a concentration of 1.25 mg/mL using a 0.5% methylcellulose solution. The anti-mouse PD-1 antibody (CD279; available from BioXCell) was diluted with PBS and prepared at a concentration of 1 mg/mL.

### (ii) Test using models with subcutaneous transplantation of MC38 cells

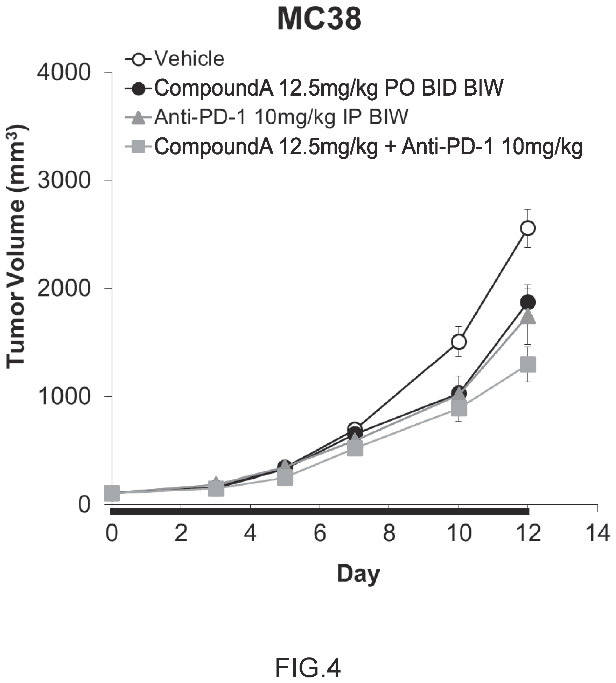

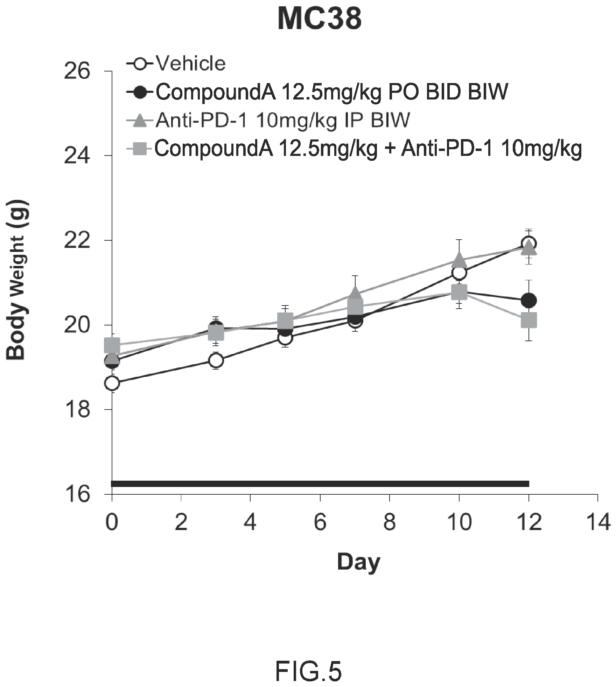

A cell suspension of cultured MC38 cells, a mouse colorectal cancer cell line, was subcutaneously transplanted into the abdominal subcutaneous tissue of female C57BL/6 mice with the age of from seven to nine weeks at 1 × 10⁶ cells / 0.1 mL PBS per mouse. The tumor volume was measured using calipers and calculated according to the formula: $Tumor volume = \left(tumor major axis\times tumor minor axis\times tumor minor axis\right) / 2 .$ When the tumor volume reached about 100 mm³, the mice were divided into four groups, G1 to G4, so that each group consisted of ten animals (G1: control group, G2: group with the administration of the Compound A, G3: group with the administration of the anti-PD-1 antibody, and G4: group with the administration of the Compound A + the anti-PD-1 antibody in combination). The group with the administration of the Compound A (G2) received oral administration (PO) of the Compound A at a dose of 12.5 mg/kg, two times daily (BID) and two times weekly (BIW) for 12 days. The group with the administration of the anti-PD-1 antibody (G3) received intraperitoneal administration of the antibody at a dose of 10 mg/kg, once daily and two times weekly for 12 days. Further, the group with combined administration (G4) received the Compound A (12.5 mg/kg) and the anti-PD-1 antibody (10 mg/kg), and the control group (G1) received oral administration of 0.5% methylcellulose solution. The amount of the drug solution to be administered was 10 mL/kg. After the grouping, the mice were measured for the tumor volume and the body weight three times per week. The measurement results of the tumor volume and the body weight are shown in Fig. 4 and Fig. 5, respectively. Further, the administration period is indicated by a thick black line.

### (iii) Evaluation of antitumor effect

With respect to the amount of change in the tumor volume during the administration period for each group and each individual, statistical analysis was carried out by two-way analysis of variance using EZR (ver. 1.54) software.

As an indicator of the antitumor effect, the T/C value (%) calculated by the following equation was used: 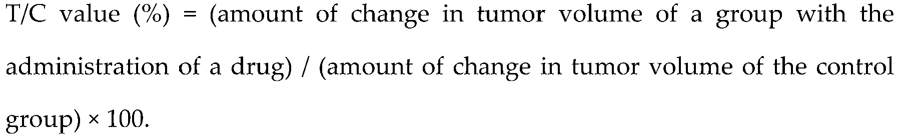 T/C value (%) = (amount of change in tumor volume of a group with the administration of a drug) / (amount of change in tumor volume of the control group) × 100.

The results are shown in Table 7 below.

**[Table 7]**

| Group | N | Tested compound | Dose (mg/kg) | Route and schedule | %BWL (Daily maximum) | Number of animals removed | Average tumor volume (mm³) day12±SEM | Average change in tumor volume (mm³) (Day 12 - Day 0) | T/C(%) |
|---|---|---|---|---|---|---|---|---|---|
| G1 | 10 | Vehicle | N/A | PO BID BIW | 0.0(0) | 0 | 2557.1±175.7 | 2454.8 | N/A |
| G2 | 10 | Compound A | 12.5 | PO BID BIW | 0.0(0) | 0 | 1874.4±156.1 | 1770 | 72.2 |
| G3 | 10 | Anti-PD-1 antibody | 10 | IP BIW | 0.0(0) | 0 | 1743.7±262.4 | 1639.4 | 66.8 |
| G4 | 10 | Compound A+ | 12.5 | PO BID BIW | 0.0 (0) | 0 | 1295.7±161.7 | 1191.3 | 48.6 |
| | | anti-PD-1 antibody | 10 | IP BIW | | | | | |

The T/C value (%) on Day 12 in the group with the combined administration (G4) was 48.6%. Thus, a stronger antitumor effect was observed as a result of using the Compound A and the anti-PD-1 antibody in combination, as compared with those of the groups to which the Compound A and the anti-PD-1 antibody were respectively administered as a single agent treatment (G2, G3).

Example 3: Combined effect of the Compound A and an antitumor agent selected from the group consisting of quizartinib, midostaurin, cytarabine, and azacitidine (combination of two agents)

In order to determine whether the combination of the pharmaceutical compositions of the present invention exhibited an additive action or a synergistic effect in the antitumor effect, the Compound A and an antitumor agent selected from the group consisting of quizartinib, midostaurin, cytarabine, and azacitidine were combined and administered in combination to mice subcutaneously bearing a tumor. The amount of change in tumor volume of the subcutaneously transplanted tumor during the administration period was compared between a control group and a group with the administration of a drug and evaluated using the effect indicator T/C. For the determination of the combined effect, the presence or the absence of an additive effect or a synergistic effect was statistically analyzed by two-way analysis of variance using the amount of change in the tumor volume of each individual.

MV-4-11 cells (human acute myeloid leukemia cell line) were purchased from ATCC, and female BALB/cA Jcl-nu/nu mice with the age of six weeks were purchased from CLEA Japan, Inc.

### (i) Preparation of drug solutions to be administered

The Compound A was prepared as a suspension at a concentration of 0.625 mg/mL using a 0.5% methylcellulose solution. Quizartinib was prepared as a suspension at a concentration of 0.03 mg/mL using 2-hydroxypropyl-betacyclodextrin. Midostaurin was prepared as a suspension at a concentration of 1 mg/mL using a 0.5% methylcellulose solution. Cytarabine was diluted with physiological saline to prepare a solution at a concentration of 2.5 mg/mL. Azacitidine was diluted with physiological saline to prepare a solution at a concentration of 0.5 mg/mL.

### (ii) Test using models with subcutaneous transplantation of MV-4-11 cells

A cell suspension of cultured MV-4-11 cells, a human acute myeloid leukemia cell line, was subcutaneously transplanted into the abdominal subcutaneous tissue of female BALB/cA Jcl-nu/nu mice with the age of six weeks at 3 × 10⁶ cells / 0.1 mL per mouse (1:1 mixture solution of HBSS and Matrigel). The tumor volume was measured using calipers and calculated according to the formula:$Tumor volume = \left(tumor major axis \times tumor minor axis \times tumor minor axis\right) / 2$

When the tumor volume reached about 130 mm³, the mice were divided into ten groups, G1 to G10, so that each group consisted of five animals (G1: control group, G2: group with the administration of the Compound A, G3: group with the administration of quizartinib, G4: group with the administration of midostaurin, G5: group with the administration of cytarabine, G6: group with the administration of azacitidine G7: group with the administration of the Compound A + quizartinib in combination, G8: group with the administration of the Compound A + midostaurin in combination, G9: group with the administration of the Compound A + cytarabine in combination, and G10: group with the administration of the Compound A + azacitidine in combination).

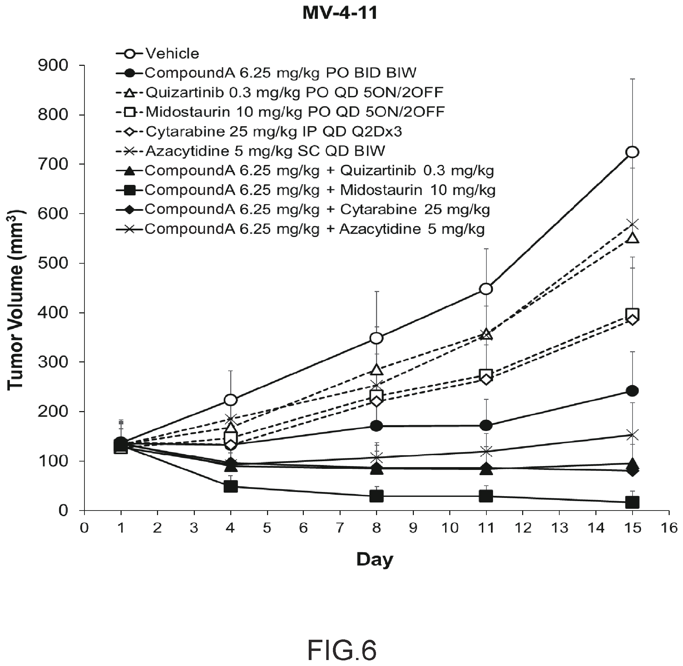

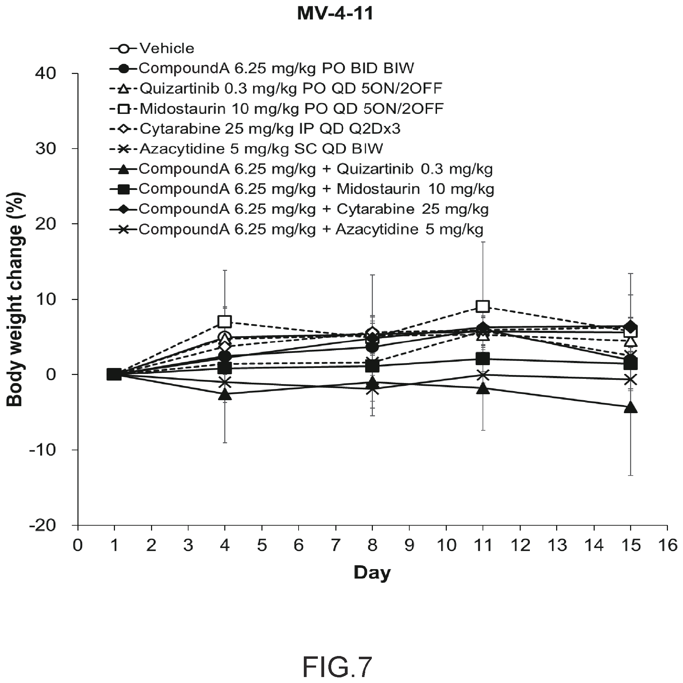

The group with the administration of the Compound A (G2) received oral administration (PO) of the Compound A at a dose of 6.25 mg/kg, two times daily (BID) and two times weekly (BIW), for 14 days. The group with the administration of quizartinib (G3) received oral administration (PO) of quizartinib at a dose of 0.3 mg/kg, once daily (QD), on a schedule of dosing five days and withdrawing two days per week, for 14 days. The group with the administration of midostaurin (G4) received oral administration (PO) of midostaurin at a dose of 10 mg/kg, once daily (QD), on a schedule of dosing five days and withdrawing two days per week, for 14 days. The group with the administration of cytarabine (G5) received intraperitoneal administration (IP) of cytarabine at a dose of 25 mg/kg once every two days (Q2D), on a schedule of three times per week, for 14 days. The group with the administration of azacitidine (G6) received subcutaneous administration (SC) of azacitidine at a dose of 5 mg/kg, once daily (QD), on a schedule of two times per week, for 14 days. The group with the combined administration (G7) received administration of the Compound A (6.25 mg/kg) and quizartinib (0.3 mg/kg). The group with the combined administration (G8) received administration of the Compound A (6.25 mg/kg) and midostaurin (10 mg/kg). The group with the combined administration (G9) received administration of the Compound A (6.25 mg/kg) and cytarabine (25 mg/kg). The group with the combined administration (G10) received administration of the Compound A (6.25 mg/kg) and azacitidine (5 mg/kg). The control group (G1) received 0.5% methylcellulose solution orally. The amount of the drug solution to be administered was 10 mL/kg. After the grouping, the mice were measured for the tumor volume and the body weight two times per week. The measurement results of the tumor volume and the body weight are shown in Fig. 6 and Fig. 7, respectively.

### (iii) Evaluation of antitumor effect

As an indicator of the antitumor effect, the T/C value (%) calculated by the following equation was used: 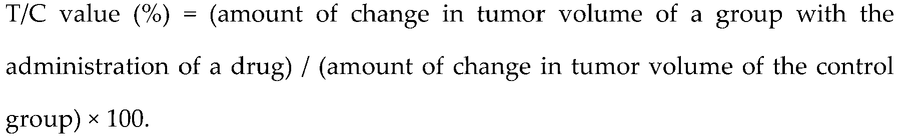 T/C value (%) = (amount of change in tumor volume of a group with the administration of a drug) / (amount of change in tumor volume of the control group) × 100.

The results are shown in Table 8 below.

**[Table 8]**

| Group | N | Tested compound | Dose (mg/kg) | Route and schedule | %BWL (Daily maximum) | Number of animals removed | Average tumor volume (mm³) day 15 | Average change in tumor volume (mm³) (Day 15 - Day 1) | T/C(%) |
|---|---|---|---|---|---|---|---|---|---|
| G1 | 5 | Vehicle | N/A | PO BID BIW | 0.0(1) | 0 | 724.2 | 587.0 | N/A |
| G2 | 5 | Compound A | 6.25 | PO BID BIW | 0.0(1) | 0 | 241.6 | 106.2 | 18.1 |
| G3 | 5 | Quizartinib | 0.3 | PO QD 5ON/2OFF | 0.0(1) | 0 | 552.2 | 418.7 | 71.3 |
| G4 | 5 | Midostaurin | 10 | PO QD 5ON/2OFF | 0.0(1) | 0 | 396.3 | 269.9 | 46.0 |
| G5 | 5 | Cytarabine | 25 | IP QD Q2D | 0.0(1) | 0 | 385.8 | 247.5 | 42.2 |
| G6 | 5 | 5-Azacytidine | 5 | SC QD BIW | 0.0(1) | 0 | 579.2 | 446.6 | 76.1 |
| G7 | 5 | Compound A+ | 6.25 | PO BID BIW | -4.2(15) | 0 | 95.6 | -40.9 | -7.0 |
| | | Quizartinib | 0.3 | PO QD 5ON/2OFF | | | | | |
| G8 | 5 | Compound A+ | 6.25 | PO BID BIW | 0.0(1) | 0 | 16.8 | -115.1 | -19.6 |
| | | Midostaurin | 10 | PO QD 5ON/2OFF | | | | | |
| G9 | 5 | Compound A+ | 6.25 | PO BID BIW | 0.0(1) | 0 | 80.6 | -54.2 | -9.2 |
| | | Cytarabine | 25 | IP QD Q2D | | | | | |
| G10 | 5 | Compound A+ | 6.25 | PO BID BIW | -1.9(8) | 0 | 153.0 | 24.6 | 4.2 |
| | | 5-Azacytidine | 5 | SC QD BIW | | | | | |

The T/C values (%) on Day 15 in the groups with the combined administration (G7, G8, G9, and G10) were -7.0%, -19.6%, -9.2%, and 4.2%, respectively. Thus, stronger antitumor effects were observed as a result of using the Compound A and the respective antitumor agents in combination, as compared with those of the groups to which the Compound A and the antitumor agents used were respectively administered as a single agent treatment (G2, G3, G4, G5, and G6).

### Example 4: Combined effect of the Compound A in combination with venetoclax and azacitidine (combination of three agents)

In order to determine whether the combination of the pharmaceutical compositions of the present invention exhibited an additive action or a synergistic effect in the antitumor effect, the Compound A and venetoclax and azacitidine as antitumor agents were combined and administered in combination to mice subcutaneously bearing a tumor. The amount of change in tumor volume of the subcutaneously transplanted tumor during the administration period was compared between a control group and a group with the administration of a drug and evaluated using the effect indicator T/C. For the determination of the combined effect, the presence or the absence of an additive effect or a synergistic effect was statistically analyzed by two-way analysis of variance using the amount of change in the tumor volume of each individual.

MV-4-11 cells (human acute myeloid leukemia cell line) were purchased from ATCC, and female BALB/cA Jcl-nu/nu mice with the aged of six weeks were purchased from CLEA Japan, Inc.

### (i) Preparation of drug solutions to be administered

The Compound A was prepared as a suspension at a concentration of 0.625 mg/mL using a 0.5% methylcellulose solution. Venetoclax was prepared as a solution at concentrations of 2.5 and 5 mg/mL using Phosal 50 PG/PEG400/Ethanol. Azacitidine was diluted with physiological saline and prepared as a solution at a concentration of 0.5 mg/mL.

### (ii) Test using models with subcutaneous transplantation of MV-4-11 cells

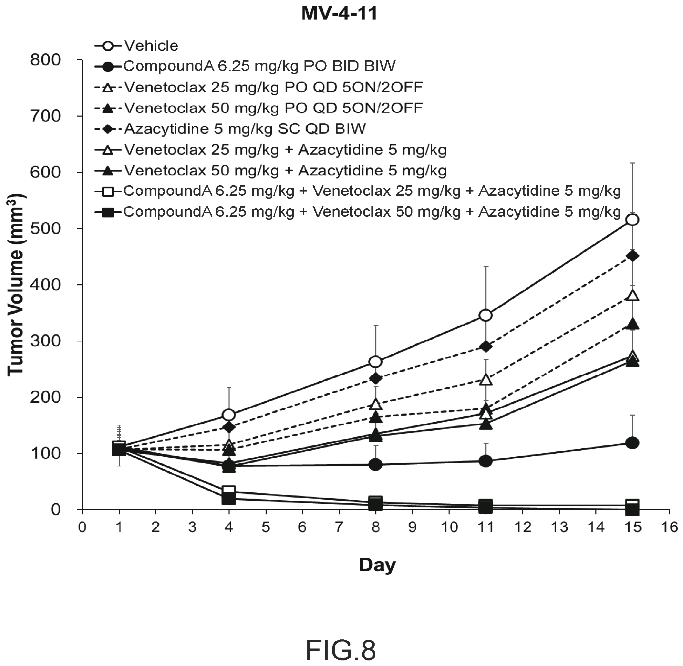

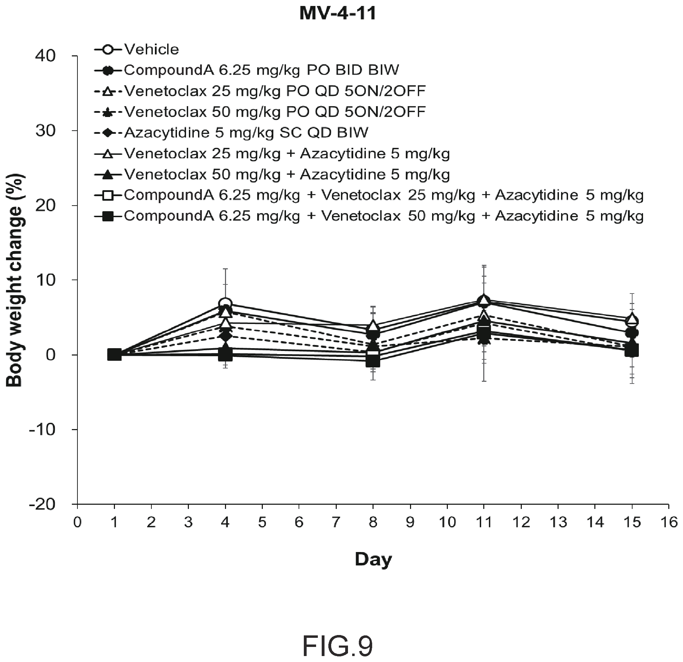

A cell suspension of cultured MV-4-11 cells, a human acute myeloid leukemia cell line, was subcutaneously transplanted into the abdominal subcutaneous tissue of female BALB/cA Jcl-nu/nu mice with the age of six weeks at 3 × 10⁶ cells / 0.1 mL per mouse (1:1 mixture solution of HBSS and Matrigel). The tumor volume was measured using calipers and calculated according to the formula: $Tumor volume = \left(tumor major axis\times tumor minor axis\times tumor minor axis\right) / 2 .$ When the tumor volume reached about 110 mm³, the mice were divided into nine groups, G1 to G9, so that each group consisted of five animals (G1: control group, G2: group with the administration of the Compound A, G3 and G4: groups with the administration of venetoclax, G5: group with the administration of azacitidine, G6 and G7: groups with the administration of venetoclax + azacitidine in combination, and G8 and G9: groups with the administration of the Compound A + venetoclax + azacitidine in combination). The group with the administration of the Compound A (G2) received oral administration (PO) of the Compound A at a dose of 6.25 mg/kg, two times daily (BID), two times weekly (BIW), for 14 days. The groups with the administration of venetoclax (G3 and G4) received oral administration (PO) of venetoclax at doses of 25 and 50 mg/kg, respectively, once daily (QD), on a schedule of dosing five days and withdrawing two days per week, for 14 days. The group with the administration of azacitidine (G5) received subcutaneous administration (SC) of azacitidine at a dose of 5 mg/kg, once daily (QD), on a schedule of two times per week, for 14 days. The group with the combined administration (G6) received the administration of the Compound A (6.25 mg/kg) and venetoclax (25 mg/kg). The group with the combined administration (G7) received the administration of the Compound A (6.25 mg/kg) and venetoclax (50 mg/kg). The group with the combined administration (G8) received the administration of the Compound A (6.25 mg/kg), venetoclax (25 mg/kg), and azacitidine (5 mg/kg), and the group with the combined administration (G9) received the administration of the Compound A (6.25 mg/kg), venetoclax (50 mg/kg), and azacitidine (5 mg/kg). The control group (G1) received 0.5% methylcellulose solution orally. The amount of the drug solution to be administered was 10 mL/kg. After the grouping, the mice were measured for the tumor volume and the body weight two times per week. The measurement results of the tumor volume and the body weight are shown in Fig. 8 and 9, respectively.

### (iii) Evaluation of antitumor effect

As an indicator of the antitumor effect, the T/C value (%) calculated by the following equation was used: 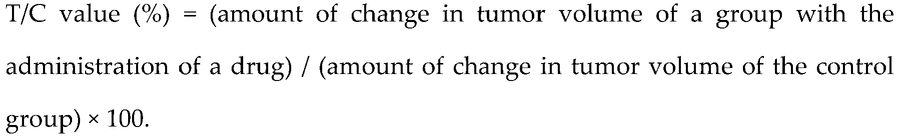 T/C value (%) = (amount of change in tumor volume of a group with the administration of a drug) / (amount of change in tumor volume of the control group) × 100.

The results are shown in Table 9 below.

**[Table 9]**

| Group | N | Tested compound | Dose (mg/kg) | Route and schedule | %BWL (Daily maximum) | Number of animals removed | Average tumor volume (mm³) day15 | Average change in tumor volume (mm³) (Day 15 - Day 1) | T/C(%) |
|---|---|---|---|---|---|---|---|---|---|
| G1 | 5 | Vehicle | N/A | PO BID BIW | 0.0(0) | 0 | 516.1 | 404.7 | N/A |
| G2 | 5 | Compound A | 6.25 | PO BID BIW | 0.0(0) | 0 | 118.5 | 7.2 | 1.8 |
| G3 | 5 | Venetoclax | 25 | PO QD 5ON/2OFF | 0.0(0) | 0 | 381.9 | 275.2 | 68.0 |
| G4 | 5 | Venetoclax | 50 | PO QD 5ON/2OFF | 0.0(0) | 0 | 331.2 | 223.2 | 55.1 |
| G5 | 5 | 5-Azacytidine | 5 | SC QD BIW | 0.0(0) | 0 | 452.2 | 344.6 | 85.2 |
| G6 | 5 | Venetoclax+ | 25 | PO QD 5ON/2OFF | 0.0(0) | 0 | 273.7 | 167.2 | 41.3 |
| | | 5-Azacytidine | 5 | SC QD BIW | | | | | |
| G7 | 5 | Venetoclax+ | 50 | PO QD 5ON/2OFF | 0.0(0) | 0 | 264.9 | 155.6 | 38.5 |
| | | 5-Azacytidine | 5 | SC QD BIW | | | | | |
| G8 | 5 | Compound A+ | 6.25 | PO BID BIW | -0.1(8) | 0 | 7.6 | -103.9 | -25.7 |
| | | Venetoclax+ | 25 | PO QD 5ON/2OFF | | | | | |
| | | 5-Azacytidine | 5 | SC QD BIW | | | | | |
| G9 | 5 | Compound A+ | 6.25 | PO BID BIW | -0.8(8) | 0 | 0.0 | -105.7 | -26.1 |
| | | Venetoclax+ | 50 | PO QD 5ON/2OFF | | | | | |
| | | 5-Azacytidine | 5 | SC QD BIW | | | | | |

The T/C value (%) on Day 15 in the group with the administration of the Compound A as a single agent treatment (G2) was 1.8%. The T/C values (%) on Day 15 in the groups with the combined administration of venetoclax and azacitidine as a two-agent treatment (G6 and G7) were 41.3% and 38.5%, respectively. Thus, stronger antitumor effects were exhibited as compared with those of the groups to which venetoclax or azacitidine was each administered as a single agent treatment (G3, G4, and G5). However, in the groups with the combined administration of the Compound A, venetoclax, and azacitidine as a three-agent treatment (G8 and G9), the T/C values (%) on Day 15 were -25.7% and -26.1%, respectively. Thus, clearly stronger antitumor effects were exhibited even as compared with those of the groups with the combined administration of venetoclax and azacitidine as a two-agent treatment. In addition, in the groups with the combined administration as a three-agent treatment (G8 and G9), complete disappearance of the tumor was observed in three out of five cases and five out of five cases, respectively.

### Example 5: Combined effect of the Compound A and an antitumor agent selected from the group consisting of niraparib, docetaxel, carboplatin, and paclitaxel (combination of two agents)

In order to determine whether the combination of the pharmaceutical compositions of the present invention exhibited an additive effect or a synergistic effect in the antitumor effect, the Compound A and an antitumor agent selected from the group consisting of niraparib, docetaxel, carboplatin, and paclitaxel were combined and administered in combination to mice subcutaneously bearing a tumor. The amount of change in tumor volume of the subcutaneously transplanted tumor during the administration period was compared between a control group and a group with the administration of a drug and evaluated using the effect indicator T/C. OVCAR-8 cells (human ovarian cancer cell line) and female BALB/c nude mice with the age of from eight to nine weeks were those prepared by the contract research organization Crown Bioscience, Inc.

### (i) Preparation of drug solutions to be administered

The Compound A was prepared as a suspension at a concentration of 1.25 mg/mL using a 0.5% methylcellulose solution. Niraparib was prepared as a solution at a concentration of 3.75 mg/mL in 10% DMSO / 40% PEG300 / 5% Tween 80/ 45% physiological saline (volume ratio). For docetaxel, 0.3 mL of docetaxel (Taxotere (registered trademark)) at a concentration of 40 mg/mL was diluted with 0.9 mL of 13% ethanol to prepare 1.2 mL of a 10 mg/mL solution, and then 1.0 mL of the solution was diluted with 4 mL of physiological saline to prepare a solution at a concentration of 2 mg/mL. For carboplatin, 10 mg/mL of a solution to be administered was diluted with physiological saline to prepare a solution at a concentration of 5 mg/mL. For paclitaxel, 6 mg/mL of a solution to be administered was diluted with physiological saline to prepare a solution at a concentration of 3 mg/mL.

### (ii) Test using models with subcutaneous transplantation of OVCAR-8 cells

A cell suspension of cultured OVCAR-8 cells, a human ovarian cancer cell line, was subcutaneously transplanted into the abdominal subcutaneous tissue of female BALB/c nude mice with the age of eight weeks at 1 × 10⁷ cells / 0.1 mL per mouse (1:1 mixture solution of PBS and Matrigel). The tumor volume was measured using calipers and calculated according to the formula:$Tumor volume = \left(tumor major axis\times tumor minor axis\times tumor minor axis\right) / 2 .$ When the tumor volume reached about 160 mm³, the mice were divided into ten groups, G1 to G10, so that each group consisted of six animals (G1: control group, G2: group with the administration of the Compound A, G3: group with the administration of niraparib, G4: group with the administration of docetaxel, G5: group with the administration of carboplatin, G6: group with the administration of paclitaxel, G7: group with the administration of the Compound A + niraparib in combination, G8: group with the administration of the Compound A + docetaxel in combination, G9: group with the administration of the Compound A + carboplatin in combination, and G10: group with the administration of the Compound A + paclitaxel in combination).

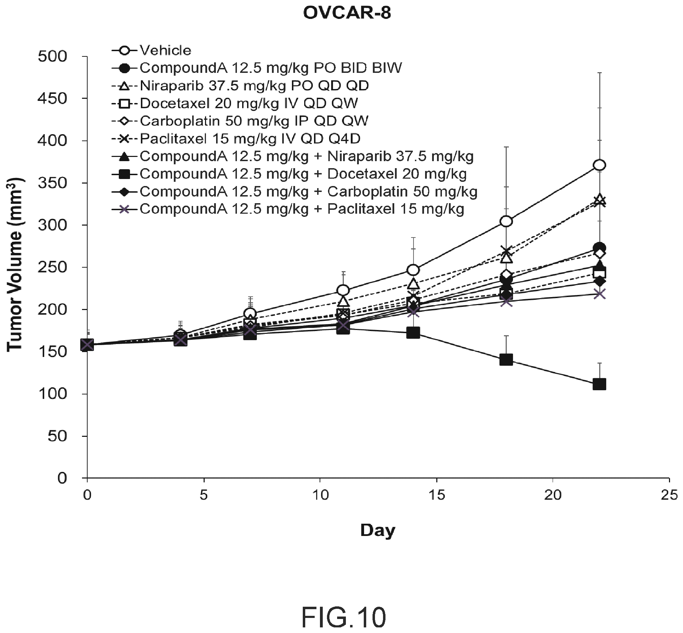

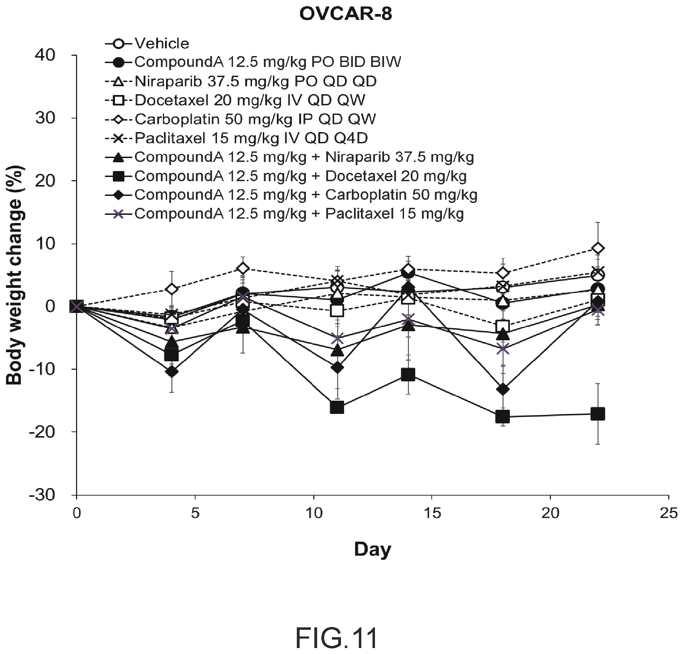

The group with the administration of the Compound A (G2) received oral administration (PO) of the Compound A at a dose of 12.5 mg/kg, two times daily (BID) and two times weekly (BIW), for 21 days. The group with the administration of niraparib (G3) received oral administration (PO) of niraparib at a dose of 37.5 mg/kg, once daily (QD) and every day, for 21 days. The group with the administration of docetaxel (G4) received intravenous administration (IV) of docetaxel at a dose of 20 mg/kg, once daily (QD), on a schedule of once weekly (QW), for 21 days. The group with the administration of carboplatin (G5) received intraperitoneal administration (IP) of carboplatin at a dose of 50 mg/kg, once daily (QD), on a schedule of once weekly (QW), for 21 days. The group with the administration of paclitaxel (G6) received intravenous administration (IV) of paclitaxel at a dose of 15 mg/kg, once daily (QD), on a schedule of once every four days (Q4D), for 21 days. The group with the combined administration (G7) received the administration of the Compound A (12.5 mg/kg) and niraparib (37.5 mg/kg). The group with the combined administration (G8) received the administration of the Compound A (12.5 mg/kg) and docetaxel (20 mg/kg). The group with the combined administration (G9) received administration of the Compound A (12.5 mg/kg) and carboplatin (50 mg/kg). The group with the combined administration (G10) received administration of the Compound A (12.5 mg/kg) and paclitaxel (15 mg/kg). The control group (G1) received oral administration of 0.5% methylcellulose solution. The amount of the drug solution to be administered was 5 mL/kg for paclitaxel and 10 mL/kg for the others. After the grouping, the mice were measured for the tumor volume and the body weight two times per week. The measurement results of the tumor volume and the body weight are shown in Fig. 10 and Fig. 11, respectively.

### (iii) Evaluation of antitumor effect

As an indicator of the antitumor effect, the T/C value (%) calculated by the following equation was used: 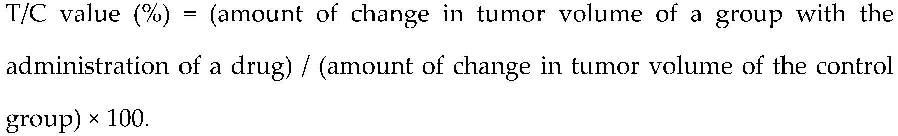 T/C value (%) = (amount of change in tumor volume of a group with the administration of a drug) / (amount of change in tumor volume of the control group) × 100.

The results are shown in Table 10 below.

**[Table 10]**

| Group | N | Tested compound | Dose (mg/kg) | Route and schedule | %BWL (Daily maximum) | Number of animals removed | Average tumor volume (mm³) day22 | Average change in tumor volume (mm³) (Day 22 - Day0) | T/C(%) |
|---|---|---|---|---|---|---|---|---|---|
| G1 | 6 | Vehicle | N/A | PO BID BIW | -2.1 (4) | 0 | 371.0 | 212.6 | N/A |
| G2 | 6 | Compound A | 12.5 | PO BID BIW | -1.7(4) | 0 | 272.7 | 113.9 | 53.6 |
| G3 | 6 | Niraparib | 37.5 | PO QD QD | -3.4(4) | 0 | 331.3 | 172.9 | 81.3 |
| G4 | 6 | Docetaxel | 20 | IV QD QW | -3.1 (18) | 0 | 243.6 | 85.3 | 40.1 |
| G5 | 6 | Carboplatin | 50 | IP QD QW | 0.0(0) | 0 | 267.0 | 108.6 | 51.1 |
| G6 | 6 | Paclitaxel | 15 | IV QD Q4D | -1.3(4) | 0 | 327.4 | 168.8 | 79.4 |
| G7 | 6 | Compound A+ | 12.5 | PO BID BIW | -6.9(11) | 1 | 252.3 | 94.0 | 44.2 |
| | | Niraparib | 37.5 | PO QD QD | | | | | |
| G8 | 6 | Compound A+ | 12.5 | PO BID BIW | -176(18) | 0 | 111.2 | 47.3 | -22.3 |
| | | Docetaxel | 20 | IV QD QW | | | | | |
| G9 | 6 | Compound A+ | 12.5 | PO BID BIW | -13.1(18) | 0 | 233.5 | 74.9 | 35.2 |
| | | Carboplatin | 50 | IP QD QW | | | | | |
| G10 | 6 | Compound A+ | 12.5 | PO BID BIW | -6.7(18) | 0 | 218.7 | 60.3 | 28.4 |
| | | Paclitaxel | 15 | IV QD Q4D | | | | | |

The T/C values (%) on Day 22 in the groups with the administration of the Compound A as a combined treatment (G7, G8, G9, and G10) were 44.2%, - 22.3%, 35.2%, and 28.4%, respectively. Thus, stronger antitumor effects were observed as a result of using the Compound A and the respective antitumor agents in combination, as compared with those of the groups to which the antitumor agents used and the Compound A were respectively administered as a single agent treatment (G2, G3, G4, G5, and G6).

### Industrial Applicability

The combination of the pharmaceutical compositions of the present invention has a remarkably higher antitumor effect, and thus is useful for the prevention and/or the treatment of cancer.

## Claims

1. A combination agent for preventing or treating cancer or a precancerous lesion, the agent comprising, in combination, a first ingredient that is a CLK inhibitor containing 1-((5-(1R)-1-fluoroethyl)-1,3,4-oxadiazolyl)methyl)-6-(4-methoxypyrrolo[2,1-f][1,2,4]triazin-5-yl)-2-methyl-1H-imidazo[4,5-b]pyridine represented by the following formula (I): 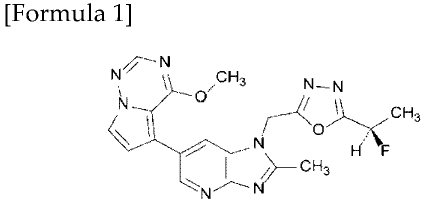 or a salt or a hydrate thereof, and a second ingredient containing at least one antitumor agent.

2. The combination agent according to claim 1, wherein the first ingredient and the second ingredient are administered simultaneously or sequentially.

3. The combination agent according to claim 1, wherein the antitumor agent of the second ingredient is selected from the group consisting of a hormonal therapeutic agent, a chemotherapeutic agent, an immunotherapeutic agent, and a molecular targeted agent.

4. The combination agent according to claim 1, wherein the antitumor agent of the second ingredient is selected from the group consisting of an antimetabolic agent, a CDK inhibitor, an anticancer antibiotic, a proteasome inhibitor, a topoisomerase I inhibitor, an ALK inhibitor, an alkylating agent, a PI3K inhibitor, an adrenocortical hormone agent, a plant-derived anticancer agent, an mTOR inhibitor, a BCL-2 inhibitor, an Akt inhibitor, a HER2 inhibitor, an EGF receptor inhibitor, a PARP inhibitor, a CSF inhibitor, a FLT3 inhibitor, an Aurora inhibitor, a MEK (MEK1/2) inhibitor, a Raf inhibitor, a PLK inhibitor, a NEDD8 inhibitor, a UAE inhibitor, a differentiation inducing agent, a cytotoxic agent, an IDH1/2 inhibitor, a VEGFR inhibitor, and an anti-PD-1 antibody.

5. The combination agent according to claim 1, wherein the antitumor agent of the second ingredient is at least one antitumor agent selected from the group consisting of 5-fluorouracil, abemaciclib, actinomycin D, all-trans retinoic acid, AZD6738, BAY1895344, bortezomib, camptothecin, ceritinib, crizotinib, cyclophosphamide, cytarabine, dactolisib, dasatinib, decitabine, dexamethasone, dinaciclib, docetaxel, doxorubicin, duvelisib, entinostat, epirubicin, etoposide, everolimus, gemcitabine, ibrutinib, imatinib, ipatasertib, tucatinib, KU-60019, lapatinib, masitinib, mercaptopurine, methotrexate, mitomycin C, mitoxantrone, MK-1775, momelotinib, navitoclax, niraparib, olaparib, palbociclib, pexidartinib, PHA-793887, quizartinib, ruxolitinib, SCH900776, SN-38, topotecan, tozasertib, trametinib, UMI-77, VE-822, vemurafenib, venetoclax, vinblastine, vincristine, volasertib, cisplatin, melphalan, oxaliplatin, EPZ-6438, JQ1, MLN-4924, MLN-7243, MPI-0479605, NMS-P715, carboplatin, paclitaxel, azacitidine, hydroxyurea, ivosidenib, enasidenib, gilteritinib, sorafenib, lenvatinib, regorafenib, midostaurin, and rucaparib.

6. The combination agent according to claim 1, wherein the antitumor agent of the second ingredient is an anti-PD-1 antibody.

7. The combination agent according to claim 1, wherein the combination agent is used for preventing or treating cancer or a precancerous lesion selected from the group consisting of colorectal cancer, lung cancer, mesothelioma, pancreatic cancer, pharyngeal cancer, laryngeal cancer, esophageal cancer, gastric cancer, duodenal cancer, small intestinal cancer, breast cancer, ovarian cancer, testicular tumor, prostate cancer, liver cancer, thyroid cancer, kidney cancer, uterine cancer, ovarian cancer, gestational trophoblastic cancer, brain tumor, retinoblastoma, skin cancer, sarcoma, malignant bone tumor, bladder cancer, acute myeloid leukemia, myelodysplastic syndrome, and other hematologic cancers.

8. The combination agent according to claim 1, wherein the combination agent is used for preventing or treating myelodysplastic syndrome, acute myeloid leukemia, or ovarian cancer.

9. A pharmaceutical composition for preventing or treating cancer or a precancerous lesion, the composition comprising a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 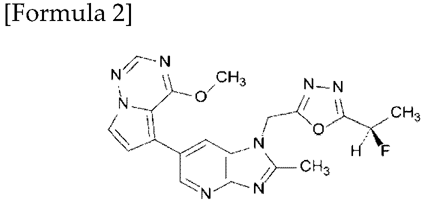 or a salt or a hydrate thereof, wherein the pharmaceutical composition is administered to a patient in need thereof simultaneously or sequentially in combination with an antitumor agent.

10. A pharmaceutical composition for preventing or treating cancer or a precancerous lesion, the composition comprising an antitumor agent, and the pharmaceutical composition being administered to a patient in need thereof simultaneously or sequentially in combination with a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 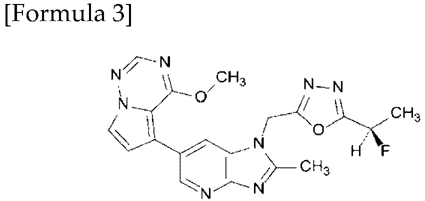 or a salt or a hydrate thereof.

11. A method for treating cancer or a precancerous lesion, the method comprising administering to a patient in need of a treatment of cancer, simultaneously or sequentially, an effective amount of a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 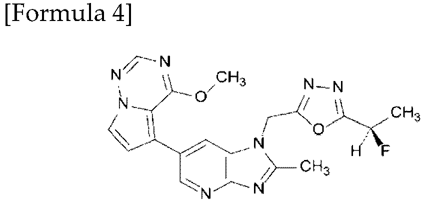 or a salt or a hydrate thereof, and an effective amount of an antitumor agent.

12. A method for treating cancer or a precancerous lesion, the method comprising administering to a patient in need of a treatment of cancer an effective amount of a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 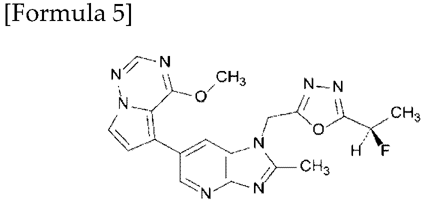 or a salt or a hydrate thereof, wherein the method further comprises administering an effective amount of an antitumor agent.

13. Use of a CLK inhibitor comprising 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 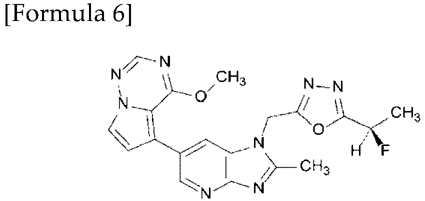 or a salt or a hydrate thereof, for manufacturing a therapeutic agent for cancer or a precancerous lesion, wherein the CLK inhibitor is administered in combination with an antitumor agent.

14. Use of an antitumor agent for manufacturing a therapeutic agent for cancer or a precancerous lesion, wherein the antitumor agent is administered in combination with a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 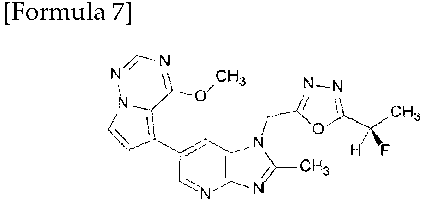 or a salt or a hydrate thereof.

15. The combination agent according to claim 1, for preventing or treating myelodysplastic syndrome, acute myeloid leukemia, or a precancerous lesion thereof, the agent comprising, in combination, a first ingredient that is a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 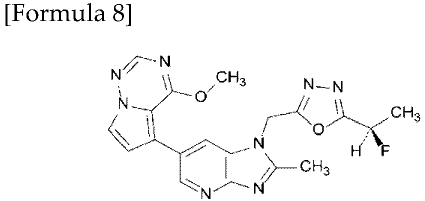 or a salt or a hydrate thereof, and a second ingredient containing at least one antitumor agent selected from the group consisting of quizartinib, midostaurin, cytarabine, venetoclax, and azacitidine.

16. The combination agent according to claim 1, for preventing or treating ovarian cancer or a precancerous lesion thereof, the agent comprising, in combination, a first ingredient that is a CLK inhibitor containing 1-((5-(1R)-1-fluoroethyl)-1,3,4-oxadiazol-yl)methyl)-6-(4-methoxypyrrolo[2,1-f][1,2,4]triazin-5-yl)-2-methyl-1H-imidazo[4,5-b]pyridine represented by the following formula (I): 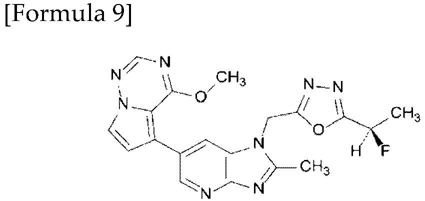 or a salt or a hydrate thereof, and a second ingredient containing at least one antitumor agent selected from the group consisting of niraparib, docetaxel, carboplatin, and paclitaxel.
